# EUROPEAN PATENT SPECIFICATION

(11) **EP 1 945 809 B1**
(45) Date of publication and mention of the grant of the patent: **27.06.2012**
(21) Application number: 06813631.6
(22) Date of filing: 16.08.2006
(51) Int. Cl.: G01N 33/00, C12Q 1/68

(54) **QUANTIFICATION OF MICROSPHERE SUSPENSION HYBRIDIZATION AND USES THEREOF**
QUANTIFIZIERUNG EINER MIKROKÜGELCHENSUSPENSIONSHYBRIDISIERUNG UND VERWENDUNGEN DAVON
QUANTIFICATION DE L'HYBRIDATION D'UNE SUSPENSION DE MICROSPHÈRES ET UTILISATIONS

(30) Priority: 16.08.2005 US 708734 P
(43) Date of publication of application: 23.07.2008
(73) Proprietor: The Children's Mercy Hospital, Kansas City, MO 64108 (US)
(72) Inventor: NEWKIRK, Heather, Kansas City, MO 64108 (US)
(74) Representative: Kalhammer, Georg
(86) International application number: PCT/US2006/032693
(87) International publication number: WO 2007/022530

(56) References cited:
- US-A- 5 510 240
- US-A1- 2003 211 604
- US-A1- 2005 003 521
- ROCKENBAUER ESZTER ET AL: "SNP genotyping using microsphere-linked PNA and flow cytometric detection" CYTOMETRY. PART A, JOHN WILEY, HOBOKEN, NJ, US, vol. 64, no. 2, 1 April 2005 (2005-04-01), pages 80-86, XP002412743 ISSN: 1552-4922
- XU HONGXIA ET AL: "Multiplexed SNP genotyping using the Qbead system: a quantum dot-encoded microsphere-based assay." NUCLEIC ACIDS RESEARCH 15 APR 2003, vol. 31, no. 8, 15 April 2003 (2003-04-15), page e43, XP002308425 ISSN: 1362-4962
- FUJA TANNIN ET AL: "A multiplex microsphere bead assay for comparative RNA expression analysis using flow cytometry" JOURNAL OF BIOTECHNOLOGY, ELSEVIER SCIENCE PUBLISHERS, AMSTERDAM, NL, vol. 108, no. 3, 18 March 2004 (2004-03-18), pages 193-205, XP002412741 ISSN: 0168-1656
- NEWKIRK HEATHER L ET AL: "Determination of genomic copy number with quantitative microsphere hybridization" HUMAN MUTATION, vol. 27, no. 4, April 2006 (2006-04), pages 376-386, XP002544643 ISSN: 1059-7794
- XIAO YAN ET AL: "Semiconductor nanocrystal conjugates, FISH and pH.", NATURE METHODS OCT 2005 LNKD- PUBMED:16179915, vol. 2, no. 10, October 2005 (2005-10), page 723, ISSN: 1548-7091
- SPIRO A ET AL: "A BEAD-BASED METHOD FOR MULTIPLEXED IDENTIFICATION AND QUANTITATION OF DNA SEQUENCES USING FLOW CYTOMETRY", APPLIED AND ENVIRONMENTAL MICROBIOLOGY, AMERICAN SOCIETY FOR MICROBIOLOGY, US LNKD- DOI:10.1128/AEM.66.10.4258-4265.2000, vol. 66, no. 10, 1 October 2000 (2000-10-01), pages 4258-4265, XP001069133, ISSN: 0099-2240
- RAO KAKUTURU V N ET AL: "Genotyping single nucleotide polymorphisms directly from genomic DNA by invasive cleavage reaction on microspheres.", NUCLEIC ACIDS RESEARCH 1 JUN 2003 LNKD- PUBMED:12771230, vol. 31, no. 11, 1 June 2003 (2003-06-01), page E66, ISSN: 1362-4962

## Description

### CROSS REFERENCE TO RELATED APPLICATION

This application claims the benefit of the prior filed, co-pending provisional application Serial No. 60/708,734, filed August 16, 2005.

### SEQUENCE LISTING

A printed Sequence Listing, hereby incorporated by reference, accompanies this application, and has also been submitted with identical contents in the form of a computer-readable ASCII file on a floppy diskette.

### BACKGROUND OF THE INVENTION

### 1. Field of the Invention

The present invention concerns materials and methods for the detection of chromosomal abnormalities using low copy nucleic acid hybridization probes. More particularly, the present invention concerns quantification of chromosomal abnormalities in nucleic acid sequences through hybridization of microsphere-conjugated, low copy nucleic acid probes to labeled target nucleic acid. Still more particularly, the present invention concerns conjugating a spectrally-encoded microsphere to a low copy or single copy nucleic acid probe, hybridizing the probe to a fluorochrome-labeled target prepared directly from subject-derived nucleic acid, detecting the product of the hybridization reaction, and quantifying the detected response by comparing the detected response with the detected response generated by a similarly hybridized reference probe.

### 2. Description of the Prior Art

Diagnosis or predisposition to human diseases often depends on the quantification of specific nucleic acid sequences in the genome or transcriptome. Current methods for detecting numerical and structural chromosomal abnormalities include chromosomal banding, fluorescence in situ hybridization (FISH), array comparative genomic hybridization (aCGH) and other microarray techniques, multiplex amplifiable probe hybridization (MAPH), multiplex ligation-dependent hybridization (MLPA), Southern analysis, and quantitative polymerase chain reaction (qPCR). Quantification of gene expression by hybridization of cDNA is typically carried out by microarray analysis (Brown et at Science 270:467-70, 1995).

Numerical abnormalities are readily detectable by chromosomal banding but structural abnormalities such as deletions or duplications need to be large (~3-5Mb) and actually disrupt banding pattern in order to be reliably detected by chromosomal banding. FISH and Southern analyses require a large quantity of patient material, involve time consuming experimental procedures, and the level of detection achieved is ~20kb to 650kb. The resolution of aCGH is limited to the size and density of cloned probes (~ 40kb for amplification of a sequence and 70-130kb for deletions) (Shaffer and Bejjani , Hum. Reprod. Update, 10(3):221-6 (2004)) and the variability of fluorescent signal can affect accuracy of interpretation (Oostlander et al., Clin. Genet., 66(6):448-95 (2004)). MAPH has a resolution of approximately 50kb (Armour et al., Nucleic Acid Res., 28(2):605-09 (2000)) and while MLPA has higher resolution than other techniques, it is time consuming, and sensitive to single nucleotide polymorphisms at or near ligation sites which can lead to false negative results. Like qPCR, care must be taken to prevent contamination (Schouten et al., Nucleic Acid Res., 30(12):e57 (2002)), and neither technique is easily multiplexed (ie. combining multiple probe sets in a single reaction).

Suspensions of spectrally-encoded polystyrene microspheres, coupled to synthetic DNA sequences, can be hybridized to a nucleic acid target sequence and detected by conventional flow cytometry as disclosed in U.S. Patent Nos. 5,736,330, 5,981,180,and 6,057,107. These assays utilize 10-50 nucleotide oligonucleotide probes for hybridization to the target DNA. Other multiplexed data acquisition and analysis platforms for flow cytometric analysis of microsphere-based assays have been disclosed (Fulton et al., Clin. Chem., 43(9):1749-56 (1997)). Standard nucleic acid hybridization assays are well known in the art and involve using a labeled nucleic acid probe to identify related target DNA or RNA molecules within a complex mixture of unlabeled nucleic acid molecules. Microsphere hybridization has been shown to be both accurate and sensitive for quantifying abundance of amplified ribosomal sequences from microorganisms in environmental samples (Spiro etal. Applied and Environmental Microbiology, 2000, p. 4258-4265, Vol. 66, No. 10). However, current assays require amplification of target DNA prior to hybridization with the probe, and current probes do not provide adequate specificity and sensitivity to accurately quantify genomic copy number directly from patient DNA samples. Polymerase chain reaction (PCR) amplification of the target DNA prior to assaying can take up to 5 hours to complete for each sequence detected, and when FISH analysis is used, the hybridization, detection and analysis process can take up to 2 days per probe per sample. Additionally, the requisite amplification step can complicate chromosomal copy number estimation of target sequences because the amplification is inherently logarithm ic and difficult to control. However, previous attempts to directly analyze unamplified genomic DNA have been unsuccessful (Borucki et al., J. Clin. Microbiol., 43(7):3255-59 (2005)).
Rockenbauer et al. (2005) Cytometry Part A 64A:80-86 describes studies on genotyping of single nucleotide polymorphisms (SNPs) using microsphere-linked peptide nucleic acid (PNA) and flow cytometric detection. To do so, genomic DNA was amplified by a two-step polymerase chain reaction (PCR).
Xu et al. (2003) Nucleic Acids Research, vol. 31, No. 8 e43 describes multiplexed SNP genotyping using a bead system. After hybridization of oligonucleotides to amplicons produced by multiplexed PCR of genomic DNA, individual microspheres were analyzed by flow cytometry.
Xiao and Barker (2004) Nucleic Acids Research, vol. 32, No. 3 e28 investigates semiconductor nanocrystal probes for human metaphase chromosomes. This reference proposes the use of semiconductor nanocrystal fluorophores in FISH (fluorescence in situ hybridization) for research and clinical applications.
Rao et al. (2003) Nucleic Acids Research, Vol. 31, No. 11 e66 describes a method for genotyping single nucleotide polymorphisms directly from genomic DNA by invasive cleavage reaction on microspheres. The microspheres are used as carriers for the DNA probe labeled with fluorescein.

The following prior art differs from the present invention, since in all methods below, prior amplification of target sample was required, and the probes coupled to microspheres were oligonucleotides which were not defined as single or low copy in all instances. Highly reiterated sequences in the genome or transcriptome do not necessarily require amplification for reliable detection, but single copy or low copy number sequences in large complex genomes do have this stipulation in order to obtain adequate sensitivity for detection. The following is a list of published and patented work involving microsphere suspension hybridization. The patents listed below are distinguished from the instant invention, since they are not enabling in terms of direct quantification of copy number of sequences in the nucleic acid target.
a. Spectrally encoded microsphere-conjugated analyte analysis: US Pats. 6,649,414, 6,449,562, 6,395,470
b. Detection of PCR and DNA using oligonucleotides: US Pats 6,057,107, 6,060,240, 5,981,180, 5,736,330, 6,916,661, and 5,981,18 8
c. Microsphere suspension array hybridization technique involving hybridization of oligonucleotide sequences to PCR-amplified DNA: US Pats. 5,736,330 & 6,057,107 and Fulton et al. Advanced multiplexed analysis with the FlowMetrix system, Clin Chem 1997 Sep; 43(9): 1749-56.
d. U.S. Pat. No. 5,981,180 discloses a method of using color-coded beads in conjunction with flow cytometry to perform multiplexed biological assay
e. Method for multiplexed analysis of DNA fragments attached to microspheres and quantification of nucleic acid using photoactivatable label attached to the target nucleic acid was patented_by Dattagupta and Nanibhushan, 2001; US Pat. 6,620,586 .
f. Patents pertaining to the attachment of oligonucleotides to microspheres, and detection of PCR products, polymorphism or methylation detection in hybridization assays follow. The following technologies may be used with that of the instant invention, but are distinct fi-om it. "Nanoparticles having oligonucleotides attached thereto and uses therefor" US Pats. 6,903,207, 6,902,895, 6,878,814, 6,861,221, 6,828,432, 6,818,753, 6,812,334, 6,777,186, 6,773,884, 6,767,702, 6,759,199, 6,750,016, 6,740,491, 6,730,269, 6,720,411, 6,720,147, 6,709,825, 6,682,895, 6,677,122, 6,673,548, 6,645,721, 6,610,491, 6,506,564, 6,495,324, 6,417,340, 6,361,944,
g. Other microsphere conjugated probe arrays for detection of particular oligonucleotide sequences: US Pats. 6,890,741, 6,858,394, 6,919,206.
h. Mutation screening using PCR for target amplification: US Pats. 6,528,261, 6,340,566 i. Mutation screening using primer extension: US Pat. 6,537,748
j. Detection of pathogens using microspheres using amplified targets: US Pats. 6,858,387, 6,620,586, 6,821,754, 6,821,519, 6,818,397

Accordingly, what is needed in the art are methods of rapidly determining the sequences of aneuploid or aneusomic domains using minute amounts of sample or patient material. What is further needed are methods for determining such sequences that are more accurate and do not require complex and time consuming steps of other current methods. What is still further needed are methods of determining numerical abnormalities of chromosomal or genomic nucleic acid sequences that do not require amplification of target DNA prior to hybridization with a probe.

### SUMMARY OF THE INVENTION

The present invention is defined in the claims.

The present invention overcomes the problems outlined above and provides a novel method for identifying chromosomal abnormalities based on measurement of genomic copy number. It may also be used to determine DNA (or RNA) concentration of a solution or to determine levels of one or more transcripts in a complex mixture of nucleic acids. Generally speaking the method of the present invention includes a microsphere suspension hybridization assay utilizing low copy genomic hybridization probes to determine copy number of a specific sequence relative to a reference sequence or standard curve. Sufficient accuracy is achieved to distinguish normal copy number which is generally two for autosomes from hemizygosity or from three or more alleles. This assay allows for the direct analysis of whole genomic DNA (or RNA) using flow cytometry and, if necessary, can follow routine cytogenetic analysis without requiring large patient sample quantities, additional blood draws, locus-specific amplifications or time-consuming genomic purification methods. It is notable therefore that copy number determination at a single locus can be carried out within a complex background of sequence consisting of the complete genome. This exquisite level of discrimination can also be used to determine copy number of rare transcripts against the background of the complete transcriptome, or for detection of extremely dilute or low concentrations of specific nucleic acid sequences within heterogeneous solutions of nucleic acids.

Unlike current hybridization assays with oligonucleotide probes, prior amplification of locus-specific target DNA is not required because the longer low copy hybridization probes of the present invention provide the increased specificity required for direct detection of homologous, unique genomic target sequences. For example, the present invention can be used to detect gains or losses in copy number in patient genomic samples with less than 90 minutes, more preferably less than one hour, even more preferably, less than 50 minutes, more preferably less than 45 minutes, still more preferably less than 40 minutes, even more preferably less than 35 minutes and still more preferably less than about 30 minutes of hands-on laboratory time, regardless of the number of probes present in each reaction per sample. Additionally, one can combine multiple independent low copy probe-conjugated microsphere sets in the same hybridization assay to independently measure multiple copy number changes. Multiplexed analysis not only decreases the amount of sample required per assay, as compared to FISH, but also decreases the analysis time and overall cost per sample tested.

In preferred forms, the present invention utilizes low or single copy hybridization probes specially designed to hybridize to a unique locus in the haploid genome sequence with high specificity. The method for probe selection and synthesis is disclosed in US Patent No. 6,828,097 and in pending U.S. Application Serial No. 09/854,867 (U.S. Patent Publication No. 2005/0064449). As can be appreciated, these initial steps require knowledge of the sequences of both the target and genomic repeats, information that is increasingly available owing to the Human Genome Project and related bioinformatic studies. Furthermore, readily available computer software is used to derive the necessary low copy sequences.

Preferably, at least two different probe sequences are selected and synthesized. At least one set of probes should be selected and synthesized for recognition of a particular nucleic acid sequence wherein the abnormality, if present, would reside (test probe), and another set of probes should be selected and synthesized for recognition of a reference sequence (reference probe). The low copy probes should be at least about 60 base pairs and generally no more than 2500 base pairs in size. Preferably, the probes are between 60 and 1000 base pairs, more preferably between about 80-500 base pairs and most preferably between about 90-1 10 base pairs. It was found that microspheres conjugated to low copy probes of about 100 base pairs produced well-defined mean fluorescence distributions and consistently higher secondary fluorochrome mean fluorescence intensity values, and thus more precisely reflected the actual copy numbers. The shorter probe conjugates are also more stable and can be used in hybridization reactions for more than two months after conjugation when properly stored, preferably in the dark and at about 4°C. This results in less lot-to-lot variation in labeled microsphere stocks, thereby reducing the effort required to conjugate, quantify and qualify probe-conjugated microspheres. Longer conjugated probes showed degraded hybridization efficiency within two weeks after conjugation.

During synthesis, the probes can be amine-tagged, depending upon the particular conjugation reaction, for conjugation to spectrally-distinct microspheres. Preferably, the probes are conjugated to the microspheres via a modified carbodiimide reaction (as described in Example 1) wherein the microspheres have been carboxylated. Other methods for coupling nucleic acids to microspheres have been developed that are equivalent in scope to the instant method. Another common method for conjugating DNA to microspheres binds strepatividin-coated beads to probes containing a modified nucleotide with a biotin moiety at the 5' terminus. Gold nanoparticles have been conjugated to thiol-modified nucleic acids in US 6361944. Protein-nucleic acids have been conjugated to beads in US 6,468,546, 6,838,243, 6,833,246,6,828,146,6,828,142. Conjugation of oligonucleotides to microspheres has also been carried out via an electrophilic tether, namely N-chloroacetamidohexyl phosphoramidite reagent (Guzaev, et al Bioorg Med Chem Lett. 1998 Dec 15;8(24):3671-6). DNA has been also conjugated to semiconductor nanocrystalline particles (Taylor, J.R., Fang, M.M., & Nie, S.M. Probing specific sequences on single DNA molecules with bioconjugated fluorescent nanoparticles. Anal. Chem. 72, 1979-1986,2000; W. Z. Guo, J. J. Li, Y. A. Wang, X. G. Peng, Conjugation chemistry and bioapplications of semiconductor box nanocrystals prepared via dendrimer bridging. Chem. Mater. 15, 3125-3133 (2003), and U.S. Patent No. 6,630,307). In a preferred embodiment, the microspheres are internally dyed, fluorescent, polystyrene beads with various spectral addresses, and are conjugated to low copy probes such that various combinations of low copy probe-conjugated beads result, wherein the reference probes are designated with a distinct spectral address. The different spectral addresses are recognized by the flow cytometer and allow for multiplexed reactions with various low copy probes attached to different microsphere sets.

The target nucleic acid sequence is then prepared for hybridization. Unlike other methods, the target sequence is not pre-selected or amplified, such that in the present invention an entire copy of a genome (or transcriptome) can be hybridized for analysis. Depending on the source and condition of the sample, the DNA (or RNA) is extracted from the cells and, if necessary, can be replicated *is vitro* using any conventional method. Preferably, the nucleic acid is replicated *in vitro* using a GenomiPhi kit (Qiagen, Valencia CA), which utilizes less than one ng of sample nucleic acid and requires less than 20 minutes hands-on time. The DNA is then labeled by any conventional means, preferably by a direct labeling step during *in vitro* replication or by an indirect labeling system consisting of a label and a reporter molecule that has an affinity for that label. The nucleic acid target is labeled with an identifying label such as a fluorophore, an enzymatic conjugate, or one selected from the group consisting of biotin or the moieties recognized by avidin, strepavidin, or specific antibodies. There are several types of non-isotopic identifying labels. One type is a label which is chemically bound to the nucleic acid target and serves as the means for direct identification. An example of this would be a-fluorochrome moiety, which upon application of radiation of proper wavelengths will become excited into a high energy state and emit fluorescent light. Directly labeled fluorescent nucleotides such as Cy3-dUTP are known in the art and would be suitable forms of labeling of target DNA for use with the instant invention. Other methods of direct labeling of DNA would also be suitable (an example would be the amino-allyl labeling marketed as the Ulysses method (Kreatech, Netherlands), however in such instances the genome DNA would have to be fragmented (by DNAse I, shearing, or other enzymatic digestion) to a suitable size for hybridization prior to addition of the labeled target to probe conjugated microspheres. In a preferred embodiment, the nucleic acid target is preferably labeled by nick translation using a modified or directly labeled nucleotide (Rigby et al., J. Mol. Biol., 113:237-251, 1977) in the conventional manner using a reactant comprising the identifying label of choice (but not limited to) conjugated to a nucleotide such as dUTP or dATP. The fragments are either directly labeled with fluorophore-tagged nucleotide or indirectly labeled by binding the labeled duplex to a fluorescently-labeled antibody that recognizes the modified nucleotide that is incorporated into the fragment as described below. Nick translations (100 µL) utilize endonuclease-free DNA polymerase I (Roche Molecular Biochemicals and DNase I (Worthington Chemical). Each fragment is combined with DNA polymerase I (4 units/microgram DNA), DNase (0.01-3 microgram/ 100 µL reaction), labeled nucleotide (0.05 mm final) and nick translation buffer. The reaction is performed at 15°C for 60 minutes and yields a variety of labeled probe fragments of different nucleotide sizes in ~300 to 1000 bp size range. Alternatively, biotin-dUTP or digoxygenin-dUTP can be incorporated during the *in vitro* replication procedure and resulting labeled sample can be treated with DNAse I or sheared by some other method to yield fragments ~300bp to 1kb in length. Other methods for labeling and detecting nucleic acids in common use may be applied to the detection of low copy DNA conjugated microspheres of the present method. These include fluorochrome labels and fluorescent compositions such as energy transfer groups, conjugated proteins, antibodies, or antigens.

The conditions for the hybridization reaction will depend upon the particular nucleotide composition and the length of each low copy probe, and are easily determined by those of skill in the art. For hybridization, the sample sequence is diluted in a hybridization buffer solution containing the low copy probe-conjugated microspheres. The amount of probe-conjugated microspheres to be utilized will depend upon the amount of sample tested. Preferably, about 5 pg to 1 µg of sample, more preferably about 25-100 ng of sample, still more preferably about 30-70 ng, yet more preferably about 40-60 ng of sample, and most preferably about 50ng of sample, is analyzed per hybridization reaction. Accordingly, the buffer solution preferably contains about 2,000-10,000 probe-conjugated microspheres, more preferably 2,000-6,000 probe-conjugated microspheres, still more preferably about 4,500-5,500 probe-conjugated microspheres, and most preferably about 5,000 probe-conjugated microspheres for each set to be hybridized. Once diluted, the hybridization reaction is heat denatured, preferably at about 95°C and then hybridized overnight at a suitable hybridization temperature, preferably, at about 45 to 51°C depending upon the probe nucleotide composition and length. The hybridized microspheres are then washed and centrifuged to remove unhybridized target sequence.

The supernatant is removed and the hybridized sample is stained or labeled with an amount of a modified reporter molecule or other suitable label, preferably one which acts as a secondary fluorochrome, to detect the labeled sample hybridized to the low copy probe-conjugated microspheres. The preferred reporter molecules are phycoerythrin-labeled streptavidin or anti-digoxigenin fluoroscein, which detect and bind the preferred target sequence labels, biotin and digoxigenin, respectively. The hybridized and labeled/stained sample is incubated at the same temperature used for the hybridization reaction, for a period of time sufficient for the reporter molecule to detect and bind the labeled target sequence. Afterwards, the sample is washed to remove residual stain. The samples are centrifuged, the supernatant removed and the stained hybridized microspheres are resuspended in an amount of hybridization buffer.

Before analysis by flow cytometry, the hybridized samples can be diluted depending upon the flow cytometer manufacturer's instructions. Preferably, about 2,000-6,000 microspheres of each set are analyzed per reaction, more preferably about 4,500-5,500 microspheres, and most preferably about 5,000 microspheres per set are analyzed per reaction. However, the amount of sample to be analyzed may depend upon the particular flow cytometer utilized for analysis. It will be appreciated that calibration and operating settings for the flow cytometer can be modified in a number of ways without undue experimentation, by those skilled in the art, to determine the optimal ranges for measuring a particular hybridization assay. These parameters will also depend upon the software employed for analysis. Fluorescent bead standards are widely available and can be used to calibrate the intensity of different fluorochrome detection channels of the flow cytometer. The instrument can also be calibrated with fluorescent reference standards based on surface-labeled beads calibrated in molecules of equivalent soluble fluorochrome (MESF) units. Photomultiplier tube voltage settings and thresholds for forward scatter, side scatter, flow rate, and various detection channels should preferably be optimized to minimize differences between fluorescence intensities of two different probes hybridized to a single patient sample with a normal genotype. Non-optimal voltage parameters are readily apparent and result in broad fluorescence peaks or non-linear data, whereas optimal parameters preferably result in tightly clustered microspheres with different spectral addresses when visualized using a side scatter plot. Preferably, these settings are determined from derived fluorescence measurements of arithmetic mean, geometric mean, median and peak channel.

It was found that most of the low copy probes of the present invention do not require significant optimization for composition, because probe quality depends mainly on the low copy nature of the sequence used to design it. Aside from methods of low copy probe design described in US #6,828,097, reference and test probe pairs were optimized for equivalent thermal stability by matching sequences of similar length, and if possible, composition (in particular, the percentage of G and C nucleotides) in an effort to mitigate secondary structure formation at or near the hybridization temperature of each probe. Sequences that are paralogous to other genomic loci produce fluorescence intensity ratios that exceed established confidence intervals for known genotypes. The present invention overcomes this limitation by use of low copy probes that are strictly not homologous to other genomic segments.

The hybridized samples are then analyzed by flow cytometry, preferably using dual laser detection, whereby the cytometer co-selects for the spectral addresses of the microspheres and the secondary fluorochrome bound to the sample sequences in order to identify and quantify the hybridized probes. Copy number differences are distinguishable by comparing the mean fluorescence intensities of the test probes with the intensities of the disomic reference probe. Specifically, the signal of each sample sequence, hybridized to its complementary probe-conjugated microsphere is determined by quantifying the fluorescence intensity of the secondary fluorochrome attached to the sample sequence. For example, the reference probe serves as an internal reference by which one copy (deletion) can be distinguished from two (normal), and two copies can be distinguished from three (duplication) based upon differences in fluorescence intensity. Compatible microsphere spectral addresses are selected to minimize overlap with the emission wavelengths of any unbound secondary fluorochrome (reporter molecule). This can be confirmed by comparison with results obtained from otherwise identical unconjugated and unhybridized microspheres. A negative control may also be maintained using a reaction tube containing all of the components except for the sample nucleic acid in order to determine background fluorescence in the secondary fluorochrome detection channel. Preferably, the system is flushed with distilled water between runs to remove any residual microspheres.

Preferably, geometric mean or median fluorescence ratio is used to measure copy number in the present hybridization assay because it provides the smallest confidence intervals (95%) and residuals for all of the genotypes with no overlap between genotypes. In sequences containing a deletion, the expected value for the geometric mean or median fluorescence ratio of test probe to reference probe intensity is about 0.5, which reflects a genomic copy number difference of one versus two. In sequences containing a duplication (or insertion), the ratio is expected to be about 1.5, which reflects a genomic copy number difference of three versus two. Ratios greater than 1.7 typically indicate increases in copy number of up to five additional copies of a sequence. And in normal sequences the ratio is expected to be about 1.0, which reflects the normal diploid copy number for both genotypes. In practice of the present invention, deviation from these expected ratios is preferably minimized, such that ratios are within a confidence interval of about 80%, more preferably about 85%, still more preferably within about 90%. In the preferred embodiment, these ratios should not be less than the 95% confidence interval and there should be no overlap between genotypes, thus allowing for a more accurate determination of copy number. Preferably ratios reflecting a deletion should be about 0.25-0.75, more preferably about 0.40-0.60, even more preferably between about 0.45-0.55, and most preferably about 0.49-0.51. Ratios reflecting either insertions or duplications should preferably be about 1.25-1.75, more preferably about 1.40-1.60, still more preferably between about 1.45-1.55, and most preferably between about 1.49-1.51. Ratios reflecting normal genotypes should preferably be about 0.75-1.25, more preferably about 0.90-1.10, still more preferably between about 0.95-1.05, and most preferably between about 0.99-1.01.

It was found that the present invention consistently distinguishes copy number differences between a diploid reference sequence and a chromosomal deletion (single allele), insertion, trisomy or duplication (three alleles), as well as increases in copy number up to five additional copies (five alleles). This assay can detect very low copy or rare sequences, even if the particular test sequence is only present in 1 copy per haploid genome. The extent of the nucleic acid rearrangement can be defined within 62 bp, which is significantly more precise than array CGH, Southern analysis and comparable to the precision of MLPA. The microsphere bead hybridization assay of the present invention improves resolution, increases signal to noise ratio, and is easily amenable to multiplexing, making it a high throughput approach. The ability to multiplex probes conserves available sample or patient material and decreases time needed to make diagnoses. Further, contamination ofheterologous genomic sequences was shown to not impact the hybridization efficiency of the assay. Consequently, the microsphere hybridization platform may be more appropriate in some diagnostic situations instead of FISH, away CGH (aCGH), expression or tiling microarray, Southern analysis, multiplex amplifiable probe hybridization (MAPH), multiplex ligation probe hybridization (MLPA) and quantitative PCR (qPCR).

This assay is easily amenable to high throughput applications. For example, with genomic always of low copy microsphere-conjugated suspension hybridization probes, it should be feasible to rapidly determine and/or define abnormal chromosomal sequences based on differences in copy number relative to one or more conserved reference loci whose copy number is essentially invariant among different individuals. Hybridization of a dense set of low copy probes that span a chromosomal region could be used to initially screen for the boundaries of aneusomic domains. Subsequent FISH studies could then provide a chromosomal context for these genetic alterations. One can envision high throughput applications, because approximately 3840 different products could be assayed on a single high-density microtiter plate using the current set of 10 spectrally distinct encoded beads in each well. With available flow cytometers, assuming a minimum probe resolution of 20kb, a single plate could provide high-resolution copy number determination for a 20Mb domain within 90 minutes of data acquisition time.

Because this invention is both more sensitive and more accurate than other art-related technologies, it may be used for other diagnostic applications (in addition to those presented in Examples 1 and 2, given herein), which have previously required either highly reiterated probes or with probes requiring prior target amplification.

One aspect of the present invention provides for detection of chromosomal imbalances. One of the most significant implications of detection of patient deletions (and possibly triallelic inheritance as well) is the resulting imbalance of alleles observed. That is, while haploinsufficiency or over expression of a transcribed genomic sequence may contribute to disease, the presence of a single mutant allele that is unmasked by a deletion of the wild type allele (or a 2:1 ratio of mutant to wild type alleles) may be as or a more important determinant of some abnormal clinical phenotypes. Judicious selection of low copy intervals may not only detect these chromosome region imbalances, but will also enable detection of mutations or SNPs that are found within these domains. Due to the efforts of the haplotype mapping consortium, detailed genomic locations and population frequencies of millions of SNPs are now available. Based on the work of some of the inventors of the present application, some of these are clearly associated with various congenital disorders.

The lc (low copy) probes used to detect chromosome imbalances using this microsphere assay can be selected based on the prior knowledge of the locations of published polymorphic sequences or mutations. After a chromosomal abnormality is detected using the assay, the hybridized microsphere is then recovered, the patient-derived sequence melted away from the conjugated sequence, the beads centrifuged down and the supernatant containing the melted strand then used in the subsequent step. This sequence could then be directly analyzed, ie. with a fluorescent PCR assay such as Taqman (Applied Biosystems) or with molecular beacon assays, or amplified and sequenced directly to determine the genotype of the hybridized allele(s). Duplexes comprised of patient samples annealed to conjugated lc probes can be trimmed to blunt ends using a nuclease (eg. S1 or Exonuclease I), then treated with Cleavase (Third Wave Technologies) to digest the DNA at mismatched sites, and the fragments separated by either dHPLC (using a Wave System, Transgenomic) or electrophoresis. Two shorter fragments whose lengths summed to the probe length would be expected (this would require conjugation of the bead to a lc probe derived from a known homozygote for the SNP - rather than from a mixture of genomic DNAs, which is the current source of template for probe synthesis).

Another alternative genotyping procedure could also use microsphere hybridization in which the recovered fragments would be used as targets in subsequent microsphere hybridization assays. The carboxylated microspheres are coupled to 5' amino labeled probes (50-70 nucleotides) in which the SNP is situated in the middle of the sequence. Since the sequence of these probes is determined by the location of the SNIP, their sequences are not necessarily low or single copy. Two distinct microspheres are employed, one coupled to the wild type sequence (reference probe), and the other containing the variant SNP sequence (test probe). Hybridization is carried out as described previously, except the hybridization time is reduced to 15 to 30 minutes due to the reduced complexity of the target sequence. The probe containing the base mismatch is destabilized by stringent washes following the hybridization and dissociates. The microsphere containing an exact sequence match will produce much stronger mean fluorescence intensity. US Pat. 6,355,431 and related patents disclose that microsphere-based hybridization can be used to initiate genotyping, however, these patents anticipate that the DNA captured by hybridization was previously obtained by target amplification. The instant invention eliminates the requirement for target amplification prior to hybridization.

At least in the case of deletions, the haplotypes oftightly linked SNPs (the phase) in the same labeled nucleic acid could be determined with this assay. The novelty here is that the mutation(s) and the deletion are effectively detected in a single assay.

Another aspect of the present invention could be utilized in livestock testing. Duplicated or deleted regions can be associated with Quantitative Trait Loci (QTLs) (Montaldo et al. Use of molecular markers and major genes in the genetic improvement of livestock. Animal Biotechnology Volume 1. No 2. August 15, 1998, 83-89), which are currently assayed by methods such as PCR and qPCR assays for cattle and sheep. Because of its high-throughput nature, the microsphere assay for QTL deletion or duplication markers using the instant invention is more suitable for use with large herds than qPCR or PCR tests. Test probes specific to a series of QTL loci can be conjugated to spectrally distinct microspheres and hybridization efficiencies of these probes can be compared to a standard reference probe, even HOXB 1 in the case of livestock species, in an effort to discern ratios above or below 1 indicating duplications or deletions of QTLs, respectively. Analysis methods are in accordance with those described above.

In another aspect of the present invention, transgenic or knockout mice (or other organisms) could be tested for successful insertion/deletion of sequences quickly in a transgenics core using the instant invention. Current methods involve PCR or qPCR, such as those described in Nature Genetics 21, 249 -251, Modeling cancer in the mouse (1999). Test probes specific to the transgenic or knocked out sequence (test probes) can be conjugated to spectrally distinct microspheres as well as a sequence in the genome adjacent to the transgenic or knocked out sequence (reference probe). Genomic DNA or complementary DNA from the transgenic or knock-out animal is then extracted and labeled as described earlier. Hybridization and detection of test and reference probes is also as previously noted. If the MFI ratio of the test to reference probe is greater than 1, then the test probe sequence has been successfully inserted into the transgenic animal. If the MFI ratio is 0.5, then one copy of the test probe sequence has been successfully knocked out or deleted from the animal genome. If the test probe fails to hybridize, this would indicate that both copies ofthe test probe sequence have been deleted from the knock-out animal tested.

Another aspect of the present invention provides for the detection of levels of genetically modified crops. PCR based methods have become one of the standard approaches for quantifying genetically modified organism (GMO) content in crops (for example see: Zimmermann A., Hemmer W., Liniger M., Luthy J., Pauli U. Lebensmittel-Wissenschaft und - Technologie, 31(7): 664-667, 1998; Studer E., Rhyner C., Lüthy J., Hübner P. Zeitschrift für Lebensmitteluntersuchung und -Forschung A. 207(3): 207 - 213, 1998; Holst-Jensen, Sissel B. Rønning, Astrid Løvseth, Knut G. Berdal. *Analytical and Bioanalytical Chemistry.* Volume 375, Number 8, 985 - 993, 2003), however these methods have some limitations. They are particularly susceptible to false positive results with low levels of contamination and at the limits of sensitivity (<2%), these methods can give variable results including false negative findings (Ahmed F., Trends in Biotech. 20:215-223, 2002).The existing qPCR assays that the USDA uses are particularly variable at the 1% threshold of detection. A commercial improvement in the reliability of GMO assays at the threshold would be valuable since the European Union set this value as their upper limit for agricultural imports. A test probe specific to the particular genetic modification can be conjugated to a spectrally distinct microsphere. A reference probe of known copy number in the particular crop genome being investigated would also be conjugated to a spectrally distinct microsphere. All experimental procedures would be as described previously. A MFI ratio of test to reference probe of 0.5 would indicate that the genetically inserted organism has inserted in one copy, a ratio of 1 would indicate two copies, and a ratio of 1.5 or greater would indicate three or more copies ofthe GMO. If the test probe fails to hybridize, this could indicate the absence of the GMO in the crop genome.

Using the methods of the present invention, the sensitivities achieved in quantification of low copy sequences in human genomic DNA are considerably better than those currently obtained using PCR for low level detection of GMO content. Furthermore the methods of the instant invention are not susceptible to false positive detection due to low level contamination, since amplification oftarget sequences is unnecessary. Based on the ability to discriminate low level copy number differences demonstrated with the instant invention, one of skill in the art would appreciate that the microsphere suspension hybridization assay would be a superior test for GMO contamination.

Another aspect of the present invention provides methods for measuring transcript levels in cDNA or in libraries relative to standard transcript equivalents or to an internal housekeeping reference gene. Current methods to detect the presence of specific transcripts in cDNA or in libraries are typically tested using qPCR (Physiol Genomics. 2003; 16(1):90-8), or PCR (Development. 1992; 116(3):555-61), or by microsphere hybridization (US Patent 6,875,568). Current methods of PCR and microsphere hybridization do not quantify transcript levels, and only detect the presence or absence of transcript. The instant invention can enable the detection and quantification of low copy or rare mRNA sequences (~5 copies per hybridization reaction) in cDNA. (Note: 5 copies was the number experimentally determined in Example 1 during the detection of sequences in a heterologous genomic environment.) Methodology used is in accordance with the methods described above.

Another aspect of the present invention provides methods for measuring changes in transcript levels in response to stimulus (environmental or chemical perturbation) or over time. Current methods are based namely on PCR (J. Immunol. 2001. Mar 15: 166(6):3663-71,) or RTPCR (Biomaterials. 2003 Jul;24(15):2561-73). A probe specific to the transcript of interest can be conjugated to microspheres and its fluorescence intensity compared to a transcript of known expression level, such as a housekeeping gene. If the transcript level increases after the stimulus, then the MFI ratio of the test to reference probe would be greater than 1. If the transcript level decreases then the ratio would be less than 1. Standard protocols would be used for the hybridization and detection.

Another aspect of the present invention provides methods for detecting chimerism or mosaicism within a mixed population of cells in a sample. Chimerism is defined as an individual or tissue sample composed of cells derived from two genetically distinct zygotes. Mosaicism is defined as an individual or tissue sample with at least 2 or more cell lines differing in genotype or chromosomal complement derived from a single zygote. For example, a X or Y chromosome specific probe from the nonpseudoautosomal region can be used to detect Chimerism, mosaicism consisting of cells from two differentiated cell lines of opposite sex (eg. XX vs XY) or of differing sex chromosome complement (i.e 45,X vs 46,XX). These probes would demonstrate difference in copy number compared to a reference cell line which is nonmosaic or nonchimeric. Using an autosomal reference probe, for example, a 45,X individual will show a mean fluorescence ratio of ~0.5 and a 46,XX individual will have a ratio of~1.0. Individuals with mosaic or chimeric constitution will have a ratio of between 0.5 and 1.0 if the chimera is of opposite sex or the mosaic is of differing sex chromosome complements. Examples of chimerism in humans include exchange of hematopoietic stem cells by dizygotic twins in utero; fusion of two zygotes into one individual in utero; and tissue or organ transplantation. Chimerism is currently detected by methods such as interphase FISH and cytogenetics in the case of sex-mismatched cell transplants in certain cancers (Bernasconi P, Cavigliano PM, Genini E, Alessandrino EP, Colombo A, Klersy C, Malcovati L, Biaggi G, Martinelli G, Calatroni S, Caresana M, Boni M, Astori C, Bernasconi C. Leukemia. 1997 Nov;11(11):1989-90; Smith A, Robson LG, Shanna P, Shaw PJ. Pathology. 1999 Feb;31(1):25-8.; NgIO, ChanKL, Shek WH, Lee JM, Fong DY, Lo CM, Fan ST. Hepatology. 2003 Oct;38(4):989-98) or resulting from pregnancy (O'Donoghue K, Chan J, de la Fuente J, Kennea N, Sandison A, Anderson JR, Roberts IA, Fisk NM. Lancet. 2004 Jul 10-16;364(9429):179-82) and by DNA polymorphism studies such as microsatellite detection in single cells (Yu N, Kruskall MS, Yunis JJ, Knoll JH, Uhl L, Alosco S, Ohashi M, Clavijo O, Husain Z, Yunis EJ, Yunis JJ, Yunis EJ. N Engl J Med. 2002 May 16;346(20):1545-52.). Mosaicism is frequently detected by methods such as FISH and cytogenetics, but it is acceptable to detect this genotype with other DNA based methods (Lomax et al., Hum Genet. 1994 Mar;93(3):243-7; Krishnamoorthy et al, J Perinatol. 1995 Jan-Feb;15(1):47-50; Chen et al., Prenat Diagn. 2004 Oct;24(10):767-73).

In another aspect of the present invention, quantification of viral or bacterial load in tissues or plasma by comparison with a reference sequence is possible. Current methods only detect the presence or absence of such bacterial or vial contaminants by ELISA, PCR, or qPCR (Comp Med 2001 Oct:51(5): 406-12. Evaluation of diagnostic methods for *Helicobacter bilis* infection in laboratory mice., Hodzic, E et al.., Proc Natl Acad Sci U S A. 2001 Nov 20;98(24):13687-92; J Clin Microbiol. 2005 Feb;43(2):716-20; Arch Dermatol Res. 2005 Feb;296(8):345-52. Epub 2005 Jan 4; J Virol Methods. Jul 7, 2005). One method for pathogen detection does involve suspension array (J Microbiol Methods. 2005 Jun 24, and US Pats 6,821,754, 6,821,519, 6,818,397, 6,790,611, 6,709,812, 6,632,607, 6,627,198), however in each of these cases, PCR amplification of the specific pathogens is required prior to hybridization. Using suspension array analysis, test probes specific to the virus or bacterium of interest can be conjugated to microspheres and tested with a reference probe, such as HOXB1.

Yet another aspect of the present invention provides for directly measuring concentrations of nucleic acids, including nucleic acids other than DNA, in solution by hybridization and comparison to sample at a known concentration using the above-described methodologies. Some current methods for RNA concentration measurement rely on hybridization to beads attached to solid surface support (Genome Research 14:2347-2356,2004), PCR, and qPCR. Patented microsphere suspension array methods designed to quantitate nucleic acids bound involve prior amplification of products (US Pats: 6,620,586, 6,812,005, 6,355,431 and 6,858,387).

Another aspect of the present invention permits determination of differences in copy number at polymorphic genomic loci to establish relatedness for forensic or genetic predisposition studies. Instability of segmental duplication of genomic sequences is common in the human genome. This phenomenon can lead to diseases (usually constitutional or congenital) that result in haploinsufficiency or increased copy number of genes within the regions flanked by these duplications. The CMT1A example of the present invention was one ofthe earliest known examples of copy number instability leading to disease.

Some unstable segmental duplications are not strictly pathogenic. A subset of copy number differences among individuals (n=119) are associated with normal variation or polymorphism in the genome (Segmental duplications and copy-number variation in the human genome: Sharp AJ, Locke DP, McGrath SD, Cheng Z, Bailey JA, Vallente RU, Pertz LM, Clark RA, Schwartz S, Segraves R, Oseroff VV, Albertson DG, Pinkel D, Eichler EE.. Am J Hum Genet. 2005 Jul;77(1):78-88. 2005). Copy variation at these polymorphic loci are not confined to a single population, suggesting that they either are recurrent genetic events, having occurred separately in multiple founders, or were present prior to the human migration from Africa.

By appropriate selection of genomic probes from segmentally duplicated intervals, the instant invention can determine how copy number genotype varies among individuals. A DNA fingerprint of an individual can be obtained by carrying out copy number determination with a sufficiently large number of such probes conjugated to spectrally distinct microspheres. The copy number polymorphism frequency for these probes is relatively high, suggesting that these genomic markers will be sufficiently informative for forensic applications of genetic identity.

These same segmental duplications represent chromosomal architectures that predispose some individuals to undergo gametic rearrangement during meiosis. Although these individuals themselves are unaffected, they would be at higher risk for offspring with segmental aneusomy in regions with predisposing chromosome configurations. Other chromosomal rearrangements, such as reciprocal, Robertsonian and jumping translocations, inversions, isochromosomes and small marker chromosomes, may be susceptible to rearrangement related to genome structure or architecture. Segmental duplicons, AT-rich palindromes (Gotter AL, Shaikh TH, Budarf ML, Rhodes CH, Emanuel BS. Hum Mol Genet. 2004 Jan 1;13(1):103-15) and pericentromeric repeats have been localized to such rearrangement breakpoints. Analysis of the products of recombination at the junctions of the rearrangements has revealed both homologous recombination and non-homologous end joining (Shaw CJ and Lupski JR, Hum Mol Genet. 2004 Apr 1;13 Spec No 1:R57-64). By appropriate selection of probes within or adjacent to segmental duplicated regions of the chromosome, it should be feasible to determine which individuals are at increased risk for gametic chromosome rearrangements that would concomitantly increase the risk of abnormal offspring.

Further, genomic rearrangements which do not involve a loss or gain in copy number, such as inversions or translocations, can be assessed using the described method. A probe specific to the 5' or 3' terminus of the inverted or translocated sequence is conjugated to microspheres and hybridized to labeled genomic DNA containing the chromosomal abnormality (eg. ABL in the case of an ABL/BCR translocation causing chronic myelogenous leukemia). Hybridized microspheres are then recovered, the patient-derived sequence melted away from the conjugated sequence, the beads centrifuged down and the supernatant containing the melted strand then used in the subsequent step. The recovered fragments are then used as targets in subsequent microsphere hybridization assays using a test probe specific to the sequence bordering but not implicated in the inversion or translocation (eg. BCR on chromosome 22). Hybridization and detection may be carried out as described previously, except the hybridization reaction will involve the new test probe (BCR) as well as the test probe from the previous hybridization (ABL). If the MFI ratio of the two test probes is approximately equal to 1, this would indicate that the translocation or inversion has successfully been identified. If the second test probe fails to hybridize, then no abnormality was detected. The MFI ratio of the second test probe compared to the first test probe should not have a ratio greater than 1. No genomic assay in the prior art utilizing suspension array can detect the presence of translocations or inversions.

Thus, one preferred embodiment of the present invention provides a method of quantifying the copy number of a target nucleic acid sequence in a genome. The method generally comprises the steps of extracting nucleic acid containing said target nucleic acid sequence from a subject, optionally replicating said nucleic acid in vitro, attaching a label to said target nucleic acid including fragmenting the target nucleic acid, preparing a particulate-conjugated, nucleic acid probe, specific for a low copy target sequence, of known sequence selected to complement said target nucleic acid sequence, hybridizing said probe to said target nucleic acid to form a hybridization reaction product, identifying said product through detection of said particulate, and quantifying said copy number of said target nucleic acid sequence through detection of said label on said product, wherein said low copy target sequence has a copy number of 10 or less. Preferably, said particulate comprises a nanocrystalline particle, still more preferably, said particulate comprises a nanosphere, even more preferably, said particulate comprises a microsphere, and most preferably, said particulate comprises a spectrally-distinct polymer microsphere. In a particularly preferred embodiment, said microsphere comprises polystyrene, and even more particularly, said particulate comprises an internally-dyed, fluorescent, polystyrene bead having a determined spectral address. In some applications, the method further comprises the step of binding a fluorochrome to a biological moiety on said microsphere surface. In other preferred applications, the general method also includes a step selected from the group consisting of conjugating said probes to said microsphere via modified carbodiimide reaction wherein said microsphere has been carboxylated and conjugating said microsphere to said probe using an electrophilic tether, wherein said tether comprises N-chloroacetamidohexyl phosphoramidite. The extracted target nucleic acid comprises DNA selected from the group consisting of genomic DNA and complementary DNA. Labeling can be done by any conventional method, including via nick translation with an identifying label, directly labeled during an *in vitro* nucleic acid replication reaction, end labeling, and random priming. Labels can be any conventional labels, including fluorophores, enzymatic conjugates, fluorophore-tagged nucleotides, fluorescently-labeled antibodies bound to antigen-bearing nucleotides, biotin-dUTP, digoxygenin-dUTP, and combinations thereof. Preferably, the nucleic acid sequence of said probe is complementary to a low copy or single copy sequence in said subject's genome. Typical response ratios of target nucleic acid sequences containing a deletion of one or more base pairs is from about 0.1 to about 0.75 in comparison to a reference probe having a normal complement of said target nucleic acid sequence. Typical response ratios of target nucleic acid sequences containing an insertion or duplication of one or more base pairs is from about 1.25 to about 1.65 in comparison to a reference probe having a normal complement of said target nucleic acid sequence. Advantageously, the method can be adapted such that multiple probes having distinct nucleic acid sequences are conjugated to particulates having one or more distinct spectral addresses and hybridized to target nucleic acids. In preferred embodiments, said particulate comprises a streptavidin-coated or carboxylated bead bound to a biotin or amino moiety at the 3' or 5' terminus of said nucleic acid probe and has a diameter between 50 nm and 1µm. Additionally, the amount of target sequence detected per 1 µg of heterologous genomic nucleic acid sequence is within the range of 50 pg to 5 ng and differences in copy number are detected with a genomic resolution of as little as 60 bp. Preferably, said quantifying step includes the step of detecting the spectral address of said product using flow cytometry. Preferred probes have a length of between 50 and 2500 bases, more preferably between 70 and about 2500 bases, still more preferably between 60 and about 1000 bases, even more preferably between 80 and about 800 bases, still more preferably between 90 and about 110 bases, and most preferably about 100 bases.

Another preferred embodiment provides a method of detecting a suspected chromosomal abnormality in subject-derived genomic or complementary nucleic acid. Generally, the method comprises the steps of preparing a spectrally-encoded, fluorescent microsphere having a first spectral address, identifying a genomic target nucleic acid probe sequence by ascertaining the nucleotide-by-nucleotide sequence of a target nucleic acid sequence wherein the abnormality is suspected to reside, synthesizing a nucleic acid target probe, specific for a genomic low copy target sequence, of known sequence according to the identified genomic target nucleic acid probe sequence, conjugating the genomic target probe to a microsphere having a first spectral address, synthesizing a reference probe selected to hybridize to a reference nucleic acid sequence having a known copy number of said genomic target nucleic acid sequence, conjugating the reference probe to a microsphere having a second spectral address, reacting the target probe with a chromosomal target sequence containing the abnormality thereby causing the target probe to hybridize to the target sequence, reacting the reference probe of known sequence with a chromosomal reference sequence containing said chromosomal target sequence and thereby causing the reference probe to hybridize to the reference sequence, detecting the hybridized target probe to ascertain the existence of the chromosome abnormality, detecting the hybridized reference probe, and quantifying the detected hybridized target probe by comparing the response of the detected hybridized target probe with the response of the detected hybridized reference probe response, without preselection or amplification of the locus specific target sequence, wherein said low-copy target sequence has a copy number of 10 or less.
Preferably, said abnormality is selected from the group consisting of differences in copy numbers of sequences within said genomic or complementary nucleic acid, duplications, deletions, inversions, transpositions, translocation, and combinations thereof. In preferred forms, said method is effective at detecting said abnormalities with a genomic resolution of as little as 60 bp. The preferred method uses flow cytometry. Preferably, said low-copy probe has a copy number of 10,9,8,7,6,5,4,3,2,1 or 0, depending upon the application or target nucleic acid sequence.

In another preferred embodiment, the present invention provides a method of direct genomic quantitation of chromosomal abnormalities via flow cytometric detection of labeled target nucleic acid sequences hybridized to microsphere-conjugated low copy genomic or complementary nucleic acid probes. Generally, the method comprises the steps of preparing a spectrally-encoded, fluorescent microsphere having a diameter between 50 nm and 1 µm, synthesizing a target probe, specific for a genomic low copy target sequence, selected to hybridize to a particular nucleic acid sequence in the target wherein the abnormality is suspected to reside, conjugating the target probe to the microsphere, hybridizing the target probe to a target nucleic acid sequence, labeling the target nucleic acid sequence, detecting the hybridized target probe via flow cytometry, and quantitating said abnormalities based on the results of said flow cytometry, wherein the nucleic acid sequence of the probe is of a length of at least 60 bases, and wherein said low copy target sequence has a copy number of 10 or less.

Preferably, the nucleic acid sequence of a probe is as defined above. Such a method is particularly preferred for detecting and quantifying an abnormality selected from the group consisting of differences in copy numbers of sequences within said genomic or complementary nucleic acid, duplications, deletions, inversions, transpositions, translocations, and combinations thereof. As with the other preferred methods, this method can detect abnormalities with a genomic resolution of as little as 60 bp.

In another preferred embodiment, the present invention provides a method of detecting chromosomal abnormalities. Generally, the method comprises the steps of preparing a hybridization probe of known sequence by coupling a spectrally-encoded, polystyrene microsphere to a synthetic DNA sequence complementary to a genomic low copy target sequence, hybridizing said probe to genomic DNA, labeling the genomic DNA, detecting the product of said hybridization by flow cytometry, wherein the nucleic acid sequence of the probe is of a length of at least 60 bases, and wherein said low copy targets sequence has a copy number of 10 or less.

Detectable abnormalities and resolution are as described above. In preferred forms of this embodiment, an additional step of comparing the results from said flow cytometry with flow cytometry results from a hybridization product having a known copy number corresponding to said abnormality. The probe is low copy or single copy. Advantageously, the DNA for this and the other preferred embodiments can be unamplified.

### Definitions

Unless defined otherwise, all technical and scientific terms used herein have the same meaning as is commonly understood by one of ordinary skill in the art to which this invention belongs.

As used herein, "a" or "an" means "at least one" or "one or more."

As used herein, "nucleic acid (s)" refers to deoxyribonucleic acid (DNA) and/or ribonucleic acid (RNA) in any form, including inter alia, single-stranded, duplex, triplex, linear and circular forms.

As used herein, the term "reference probe" means a probe specific for a locus in the genome, preferably from an autosomal sequence, that is severely damaging and preferably lethal in any other copy number but 2. The reference probe may be derived from any low or single copy chromosomal locus, so long as it has a normal chromosomal complement in the patient sample. In determination of genomic copy number for diagnosis of constitutional disease, reference probes will typically be of autosomal origin from one or more genes that are required to be expressed from two alleles during normal development. For determination of genomic copy number for diagnosis of neoplastic disease, reference probes are selected from chromosomal domains with a paucity of oncogenes and which have normal chromosomal complement.

As used herein, "label" refers to any chemical group or moiety having a detectable physical property or any compound capable of causing a chemical group or moiety to exhibit a detectable physical property, such as an enzyme that catalyzes conversion of a substrate into a detectable product. The term "label" also encompasses compounds that inhibit the expression of a particular physical property. The "label" may also be a compound that is a member of a binding pair, the other member of which bears a detectable physical property. Exemplary labels include mass groups, metals, fluorescent groups, luminescent groups, chemiluminescent groups, optical groups, charge groups, polar groups, colors, haptens, protein binding ligands, nucleotide sequences, radioactive groups, enzymes, particulate particles and a combination thereof.

As used herein, "sample" refers to anything that may contain an target nucleic acid to be analyzed. The sample may be a biological sample, such as a biological fluid or a biological tissue. Examples of biological fluids include urine, blood, plasma, serum, saliva, semen, stool, sputum, cerebral spinal fluid, tears, mucus, amniotic fluid, or the like. Biological tissues are aggregates of cells, usually of a particular kind together with their intercellular substance that form one ofthe structural materials of a human, animal, plant, bacterial, fungal or viral structure, including connective, epithelium, skin, muscle and nerve tissues. Examples of biological tissues also include organs, tumors, lymph nodes, arteries and collections of individual cell(s), for example, isolated from plasma, blood or urine or by collagenase treatment of solid tissues.

As used herein, "amplification" refers to a method for exponentially duplicating a target analyte nucleic acid in a sample to improve assay sensitivity. As described herein, many different methods for amplifying nucleic acids are known in the art.

As used herein, "set" refers to a collection of microspheres harboring an identical spectral address conjugated either with a single lc probe or a collection of Ic probes.

As used herein, "low copy" or "Ic" refers to a sequence which will hybridize to ten or fewer sequence intervals in the target nucleic acid or locations in a genome. It is preferred that the copy number be 10 or fewer, more preferably 7 or fewer, still more preferably 5 or fewer, and most preferably 3 or fewer.

As used herein, "single copy" or "sc" refers to a nucleic acid sequence which will hybridize to three or less sequence intervals in the target nucleic acid or locations in a genome. Thus, the term will encompass sequences that are strictly unique (i.e., sequences complementary to one and only one sequence in the corresponding genome), as well as duplicons, and triplicons.

As used herein, "highly reiterated" means present in more than 10 copies.

### BRIEF DESCRIPTION OF THE DRAWING FIGURES

Fig. 1 is a schematic of a low copy probe-coupled microsphere hybridization assay;
Fig. 2 is a comparison of geometric mean ratios (x-axis) for pairs of low copy test probes (16-1 b, 16-2b, PMP22, TEKT3) to reference probe (HOXBIc) compiled from 170 reactions;
Fig. 3 contains histograms of the fluorescence detection of multiplexed low copy probe-microspheres hybridized to samples A) intact for 16-2b (Sample 47, 16-2b:HOXB 1 ratio = 0.86, Table 2), B) duplicated for TEKT3 (CMT1A-1, TEKT3:HOXB1 ratio = 1.40, Table 2) and C) deleted for 16-1b (Sample 33, 16-16:HOXB1 ratio = 0.49), and using HOXB1 as the internal genomic reference probe for each sample;
Fig. 4 is a map of PWS/AS Region on Chromosome 15q11-13; and
Fig. 5 is a summary of all QMH MFI ratios obtained in Example 2.

### DETAILED DESCRIPTION OF THE PREFERRED EMBODIMENTS

The following example sets forth preferred embodiments of the present invention. This example demonstrates detection of hemizygosity, trisomy, duplication, and insertion in the human genome and quantitation of nucleic acids in solution. However, this example is for illustrative purposes only and the disclosure herein should not be construed as a limitation upon the scope of the present invention.

### Example 1

### Materials and Methods

### Selection, coupling and hybridization of microsphere-conjugated probes

*Probe selection.* Five 1c probes were developed (Rogan et al, 2001; Knoll and Rogan, 2004) and used in this study to distinguish genomic copy number differences in the patient samples. They include: (i/ii) chromosome 9q34 probes (16-1 and 16-2) from within intron 1b of *ABL1* and deleted in a subset of chronic myelogenous leukemia (CML) patients, (iii/iv) chromosome 17p12 probes recognizing *TEKT3* and *PMP22* which are within the CMT1A duplicated region (Inoue et al, 2001), and (v) a reference chromosome 7p15 probe recognizing *HOXB1* (2 copies per diploid genome). The probe lengths, hybridization temperatures, and genome coordinates are listed in Table 1. Each probe had a copy number of one as determined for the entire length of the probe found in the genome; however, some homologous low copy sequences were found within small (<200 bases) segments of the probes.

*Low copy (lc) probe synthesis.* A single primer of each 1c probe-specific pair (22-24 nucleotides in length) was synthesized with a 5'amino-modifier C-12 for coupling to microspheres (Integrated DNA Technologies, Coralville, Iowa). Polymerase chain reaction (PCR) using Pfx (Invitrogen, Carlsbad, California) was performed with each primer pair (modified and unmodified) using control human genomic DNA (Promega, Madison, Wisconsin) as the template for Ic probe synthesis. PCR products were separated by electrophoresis in low EEO agarose (Seakem, FMC Bioproducts, Rockland, Maine) and extracted by micro-spin column centrifugation (Qiagen, Valencia, California). Products were quantitated using a spectrophotometer and subsequently conjugated to microspheres with an average of 120,000 DNA molecules coupled to each microsphere.

*Coupling of probes to microspheres.* Fluorescent microspheres, each with distinct spectral addresses (designated R1-R9; Molecular Probes, Eugene, Oregon) and coated with approximately 200,000 carboxy-sites, were conjugated individually to different lc probes. Purified amino-modified lc probes were coupled to the carboxylated microspheres via a modified carbodiimide coupling procedure (Dunbar et al, 2003; Fulton et al, 1997). Each probe was initially heat denatured and then snap-cooled on ice. Approximately 3.125 x 10⁵ microspheres with identical spectral characteristics were pipetted into a 1.5 mL microcentrifuge tube (USA Scientific, Ocala, Florida), centrifuged for 2 minutes at 10,000 g, and drained of supernatant. 150 uL of O.11VI MES buffer (2-(*N*-morpholino)ethanesulfonic acid) pH4.5 was added to each tube and the microspheres were vortexed briefly followed by centrifugation for 2 minutes at 10,000 g. The supernatant was removed and the microspheres were resuspended by vortexing in 80 µL of 0.1M MES. A single 1c probe (0.5 nmol) was added to each tube and mixed by vortexing. A 1.25 µL volume of fresh 10 mg/ml solution of 1-ethyl-3-3-dimethylaminopropyl carbodiimidehydrochloride (EDC) was added and the reaction was vortexed briefly and incubated in the dark for 30 minutes with occasional mixing. Mixing and incubation of EDC was repeated twice, using 1.25 µL of freshly prepared EDC solution each time. The reaction was stopped by the addition of 500 µL 0.02% Tween20 followed by vortexing and centrifugation for 2 minutes at 10,000 g. Following removal of the supernatant, 250 µL of 0.1 % SDS was added to each tube, vortexed, and then centrifuged at 10,000 g for 2 minutes. The supernatant was carefully removed, 25 µL of 0.1M MES pH4.5 was added, and the tube was vortexed and stored in the dark at 4°C. Coupled microsphere concentrations were quantitated by adding 1 µL of each microsphere to 100uL of 1X PBS and analyzing on the FACSCalibur flow cytometer (Becton Dickinson, San Jose, California) using conditions given below.

### Genomic DNA template preparation

Genomic DNA template was prepared from methanol-acetic acid fixed cell pellets derived either from patient samples remaining after clinical cytogenetic characterization or from cell lines obtained from the NIGMS-Coriell Cell Repository (Camden, New Jersey). The fixed cells were washed twice with 1X PBS and their concentrations were determined with a hemocytometer. Genomic DNA template was extracted from ~600 fixed cells per sample. This DNA was replicated *in vitro* using the GenomiPhi kit (Qiagen, Valencia, CA), and then nick-translated (1 µg) with biotin-16 dUTP for 60 minutes at 15°C to obtain labeled products of 300 bp to 1 kb in length (Knoll and Lichter, 1994). 50 ng of nick-translated patient sample was analyzed in each hybridization assay.

The samples utilized to validate the method were known to exhibit differences in copy number for chromosome 9q34 and chromosome 17p12 based on routine cytogenetic analysis and/or fluorescence in situ hybridization (FISH). The cytogenetic findings for these test samples are listed in Table 2.

The samples included five from patients with CML, of which two had a concerted chromosome 9q34 *ABL1* deletion (found in 10% of patients; Sinclair et al, 2000) in addition to the characteristic chromosome 9;22 translocation (designated Samples 33, 81) and three had only the chromosome 9;22 translocation with no deletion (designated Samples 38, 47, and 86); two from cell lines with complete trisomy 9 (GM09286 and GM10186); one from a cell line with a chromosome 9q34 duplication (GM06074); and 5 from patients with Charcot-Marie-Tooth Disease type 1A and a chromosome 17p12 duplication (designated CMT1A-1 through 4, and GM12214). Institutional Review Board approval was obtained for use of the residual fixed cells.

*Hybridization reactions.* For hybridization, 50 ng of each sample was diluted in 40 µL TMAC hybridization buffer (3-mol/L tetramethylammonium chloride, 50mmol/L Tris-HCl, pH8.0, 1g/L sarkosyl) containing 5,000 1c probe-coupled microspheres. The hybridization reactions were heat denatured at 95°C for 3 minutes and then hybridized overnight at 45°C to 51°C depending upon the probe nucleotide composition and length (Lewin, 1980) (Table 1). The hybridized microspheres were then washed with 250uL of 1.5X TMAC (Dunbar et al, 2003) followed by centrifugation at 10,000 g for 2 minutes. The supernatant was removed and 12 µL of a 1:50 dilution of a reporter molecule, streptavidin-phycoerythrin (SPE; Molecular Probes), in 1.5X TMAC was added to detect genomic targets containing biotin. The reactions were incubated at their hybridization temperature for 12 minutes. Following labeling, 250 µL of 1.5X TMAC was added to each reaction, mixed, and centrifuged at 10,000 g for 2 minutes. The supernatant was removed and the hybridized microspheres were resuspended in 70 µL of 1.5X TMAC.

### Flow cytometry detection of hybridized microsphere-coupled low copy probes.

Hybridization reactions were diluted in 300 µL 1X PBS prior to analysis on a FACSCalibur flow cytometer. Approximately 5,000 microspheres of each set were analyzed per reaction. The signal of each target, hybridized to its complementary probe coupled to microspheres, was determined from the fluorescence intensity of SPE. Compatible microsphere spectral addresses selected to minimize overlap with the emission wavelengths of phycoerythrin (PE) were confirmed by comparing results obtained with otherwise identical unconjugated and hybridized microspheres. For each reaction, a reaction tube with all the components except target DNA was used as a negative control to determine background fluorescence in the FL2 (PE) detection channel. Fluorescent bead standards (LinearFlow Flow Cytometry Intensity Calibration Kit; Molecular Probes) were used for intensity calibration of different fluorochrome detection channels of the flow cytometer. The instrument was also calibrated with fluorescent reference standards (Quantum R-PE MESF Medium Level Kit; Bangs Laboratories, Fishers, Indiana), based on surface-labeled beads calibrated in molecules of equivalent soluble fluorochrome (MESF) units.

Optimal photomultiplier tube voltage settings were determined by selecting photomultiplier voltage tube settings that minimized differences between fluorescence intensities of two different probes hybridized to a single patient DNA sample with a normal genotype. These settings were determined from instrument-derived fluorescence measurements (CellQuest; Becton Dickinson) of arithmetic mean, geometric mean, median and peak channel. Typical photomultiplier tube voltage settings for the FACSCalibur instrument were FSC (forward scatter) = E00 (no signal amplification), SSC (side scatter) = 344 V, FL1= 727 V, FL2 = 640 V, FL3 = 300 V, and FL4 = 500 V. Thresholds for FSC, FL1, FL2, and FL3 were set at the default of 52 V. The FSC threshold was selected as the primary parameter and had a value of 52 V and the secondary parameter was set at SSC with a value of 125 V. The flow rate was set on low and the sheath fluid used was FACsFlow (Becton Dickinson). The system was flushed between runs with 2-5 mL of distilled water to remove any residual microspheres. CellQuest was used for data collection and analysis. Analysis of data was also performed using WinMDI2.8 flow cytometry package (WinMDI; J. Trotter, Salk Institute, La Jolla, California).

Most of the probes tested did not require significant optimization for composition because probe quality depended mainly on the low copy nature ofthe sequence used to design it. The fluorescence intensity ratios fell outside of established confidence intervals or known genotypes for sequences that were paralogous to other genomic loci (data not shown). This was circumvented by using Ic probes that were strictly not homologous to other genomic segments. The optimal voltage settings resulted in tightly clustered microspheres with different spectral addresses, as visualized with CellQuest using the sidescatter (SSC) plot. Non-optimal voltage parameters resulted in broad FL2 peaks or non-linear data (Bagwell et al, 1989; Brown et al, 1994).

We first showed that all probes could detect the corresponding PCR product in a hybridization reaction, thereby establishing that probe conjugation and hybridization to microspheres were efficient. Purified *ABL1* (16-1, 16-2), *TEKT3*, *PMP22*, and *HOXB1* products were amplified from normal genomic DNA (Promega) using a single 5' biotinylated primer. The biotinylated PCR products were hybridized to complementary microsphere-conjugated probes and detected with SPE. Hybridized microsphere count and mean fluorescence intensities were measured using CellQuest software (Becton Dickinson). The density of hybridized 1c products for a set of microspheres was estimated by comparing the quantity of single beads (from the gated side scatter bead count) to the number of hybridized beads (gated bead signal count and mean fluorescence intensity level), which determined the conjugation efficiency of the carbodiimide coupling procedure. The specificity ofthe microsphere-conjugated 1c probe to its target sequence was determined by comparing mean fluorescence intensity of repeated flow cytometry runs of independent hybridization assays with homologous versus heterologous probes. Hybridization of each probe was optimized across a range of annealing temperatures (45°C to 60°C).

### Verification ofprobe specificity and sensitivity

*Genomic reconstruction experiments.* To test the specificity of 1c probes in a complex genomic environment, 1c probe-coupled microspheres were hybridized to their corresponding purified PCR products in the presence of excess sheared calf thymus DNA (Amersham Biosciences, Piscataway, New Jersey). Independent hybridization reactions were performed for 16-1 a, 16-2a and HOXB1a and were carried out with 10,000 microspheres for each probe, 5 ng of the corresponding PCR product, and 50 ng of nick-translated calf thymus genomic DNA. To estimate the lowest detectable amount of hybridized product, a dilution series of PCR products (5-150 genomic equivalents per hybridization reaction) were added to 10 ng of sheared calf thymus DNA with a molar ratio of PCR product:calf thymus DNA ranging from~1:300 to 1:10.

Multiplex hybridizations with Ic probe-conjugated microspheres for 16-1a, 16-2a, and HOXB1a and 5 ng each of their corresponding PCR products were also performed in the presence or absence of 10ng of sheared calf thymus genomic DNA. Additionally, microsphere swap experiments were performed in which 1c probe 16-1a was conjugated to two microsphere sets with different spectral addresses (R2 and R9), hybridized to corresponding PCR products in a multiplex reaction, and mean fluorescence intensity levels were compared for each microsphere set.

Statistical analysis was performed using MATLAB (MATHWORKS, Natick, Massachusetts). Mean fluorescence ratios between test and reference probes are reported using arithmetic means in text (Coder et al, 1994). Residuals were calculated by fitting mean fluorescence ratios and peak channel values to expected values based on known genotypes.

### Results

### Specificity of the hybridization assay

PCR products homologous to 1c probes coupled to microspheres were used as target DNA in hybridization reactions to examine the labeling efficiency of the carbodiimide coupling procedure. Based on the SPE mean signal (in the FL2 channel) above background fluorescence (>10¹) and the number of microspheres hybridized to PCR products present in the reaction, 98% (+/-0.4%) of the microspheres had hybridized to target PCR product (data not shown).

Genomic reconstruction experiments confirmed that 1c probes 16-1 a,16-2a, and TEKT3 are not homologous (>75%) to any sequence in the calf (*Bos taurus*) genome. Based on sequence analysis, the *Bos taurus* genome should not contain sequences closely related to probes 16-1, 16-2, TEKT3 and PMP22. Mean fluorescence intensities below background levels were consistently observed for these SPE signals. By contrast, HOXB 1a showed a mean fluorescence intensity of 12.81 in one hybridization assay, which was expected since this probe is 90% homologous to *HOXB1* sequences in the *Bos taurus* genome.

Conjugated lc probes 16-1a and 16-2a detected homologous sequences seeded into a heterologous genomic background. The sensitivity of detection was linearly related to the amount of probe present, based on increases in SPE mean fluorescence intensity with product concentration (data not shown). Microsphere swap experiments showed that these results were independent of the spectral address of the microsphere. The mean hybridization signal intensities for different microsphere intensities conjugated to the same probe gave very similar values in signal intensity. For example, for Sample 47, multiplex hybridizations of probe 16-1a conjugated to microsphere R2 yielded a mean fluorescence intensity of 32.8, and microsphere R9 gave a mean fluorescence signal of 32.02, a difference of ~2%. With Sample 33, the mean fluorescence of this same probe varied by ~13% (41.88 vs. 36.16) for microspheres with different spectral addresses.

### Optimization of probe length

Probes of varying lengths were conjugated to microspheres to examine the effect of probe length on the hybridization signal. Lc probes 16-2a (1381 bases) and 16-2b (101 bases), which are subsets of probe 16-2, were coupled to the surface of spectrally distinct microspheres and multiplexed in a single hybridization reaction to Sample 47 (which has a normal genotype at the 16-2 locus). Surprisingly, the mean fluorescence signal ratio for the shorter 16-2b probe to HOXB1c was approximately double the corresponding 16-2a ratio. A multiplex experiment with DNA from a chromosome 9 deletion patient (Sample 33) showed 16-2a and 16-2b probes to have lower signals - 52% and 66%, respectively than the HOXB1c reference. Since the deletion patient has half the number of copies of 16-2 compared to HOXB 1 c, the signal obtained with the shorter 16-2b probe appears to more precisely reflect the actual copy number in these individuals. Similar results were obtained with subsets of probe 16-1 (16-1a [2304 bp] and 16-1b [100 bp]) conjugated to microspheres when independently hybridized to the corresponding PCR product and control genomic DNA. Both probes successfully hybridized to both PCR product and control genomic DNA, however the SPE fluorescence distributions for the 16-1a reactions were much broader than they were for 16-1b. We found that microspheres conjugated to the shorter probes (∼100 bases) produced well-defined mean fluorescence distributions and consistently higher PE values. These conjugates could also be used in hybridization reactions for more than two months after conjugation when stored at 4°C in the dark, unlike longer conjugated probes which showed degraded hybridization efficiency within 2 weeks of preparation.

Shorter oligonucleotide probes (HOXBle and 16-1c) proved to be adequate for characterizing genotypes of known samples. Such probes produced test to reference probe MFI ratios that could distinguish copy number. For instance, DNA from the trisomy 9 cell line, GM09286, was hybridized with test probe 16-1c (62 bases) and reference probe HOXB1e (62 bases), yielding a MFI ratio of 1.28. This MFI ratio is consistent with the presence of 3 copies of the 16-1c sequence.

### Detection of reduced genomic copy number in samples with a deletion of 9q34

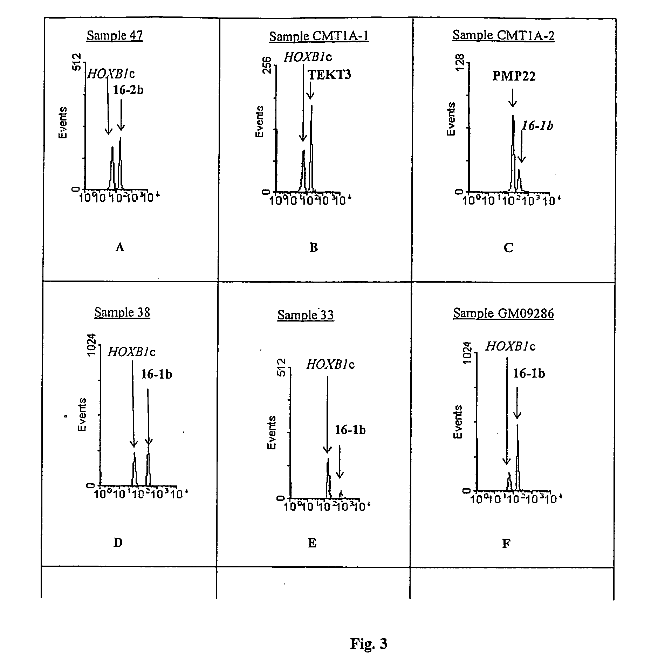

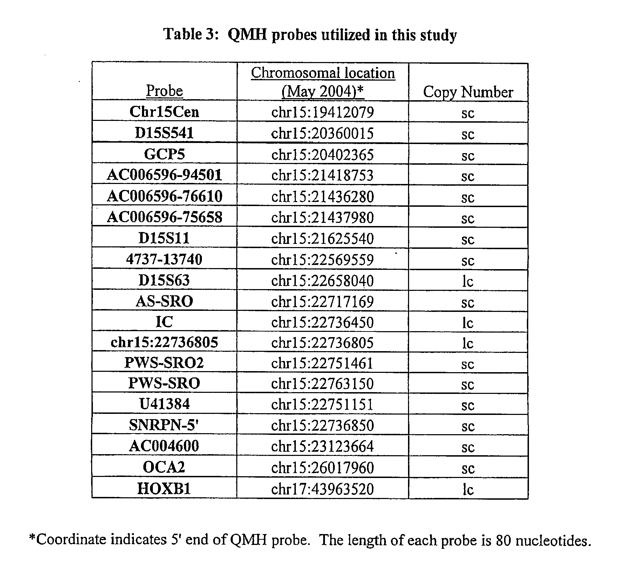

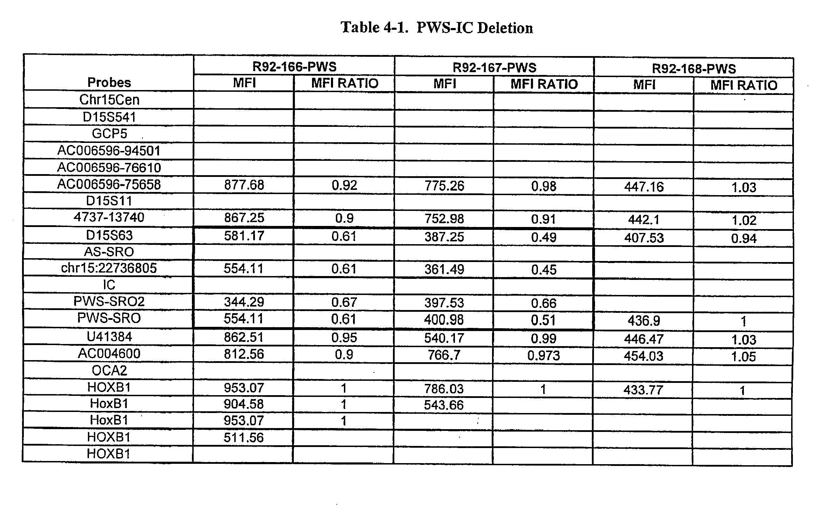

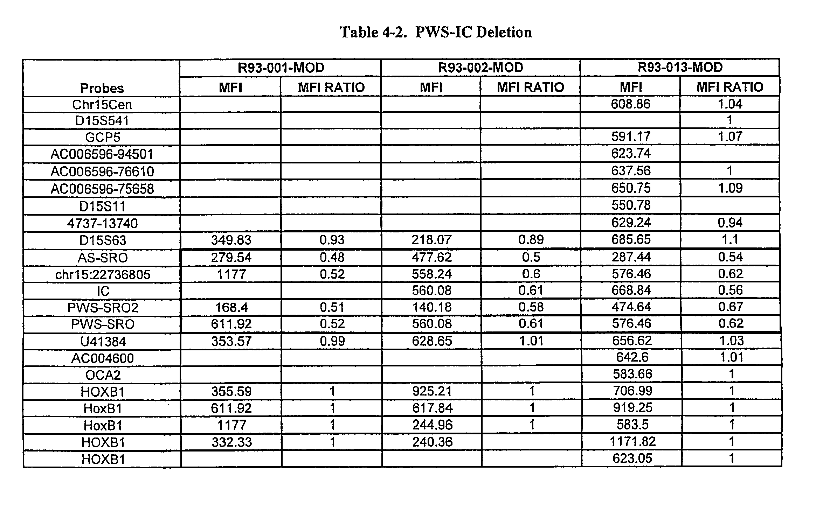

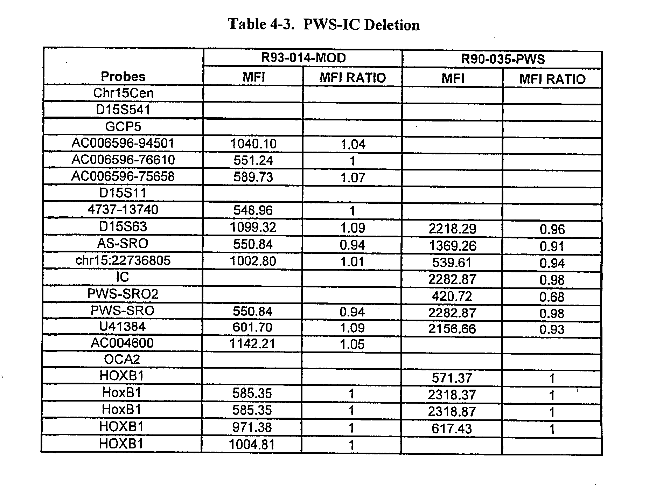

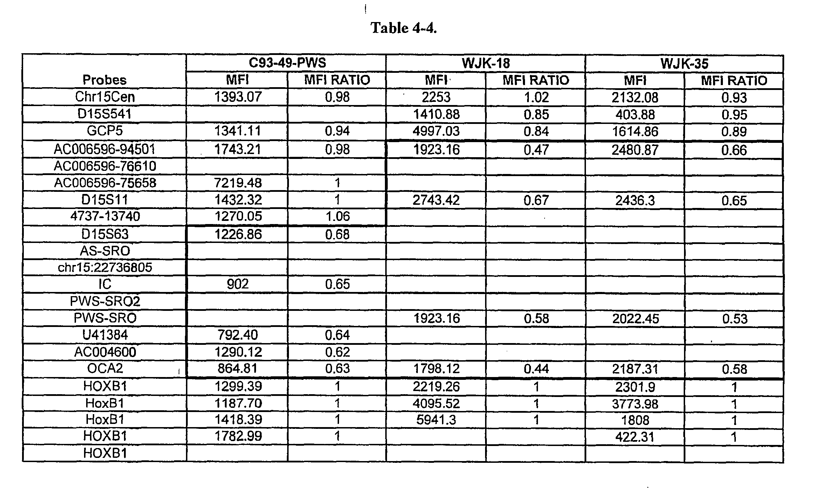

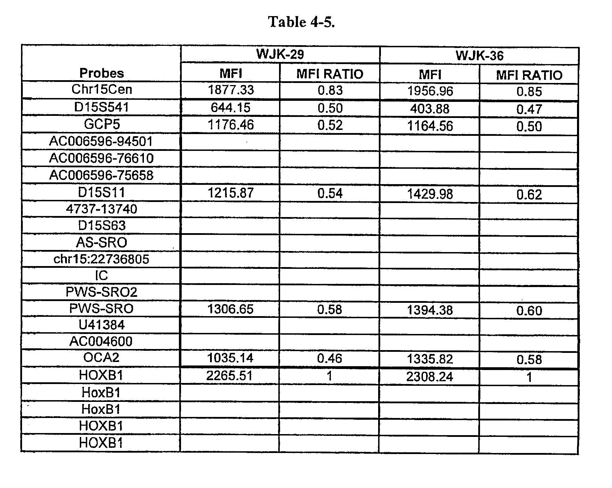

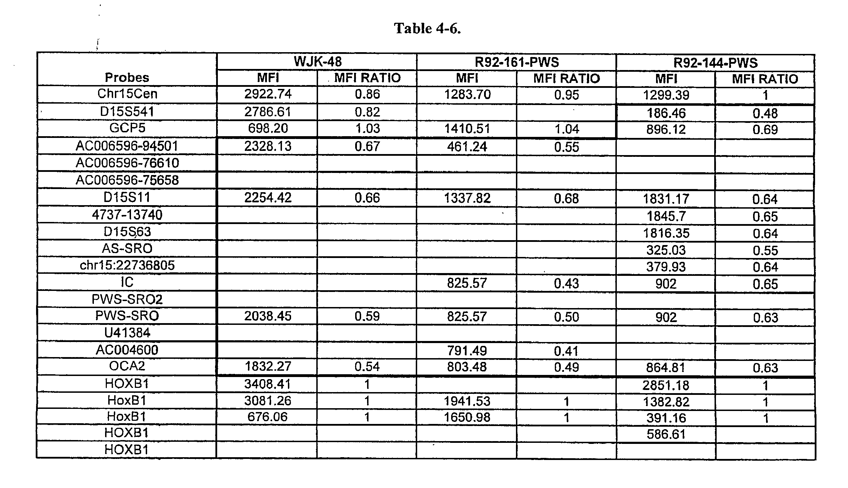

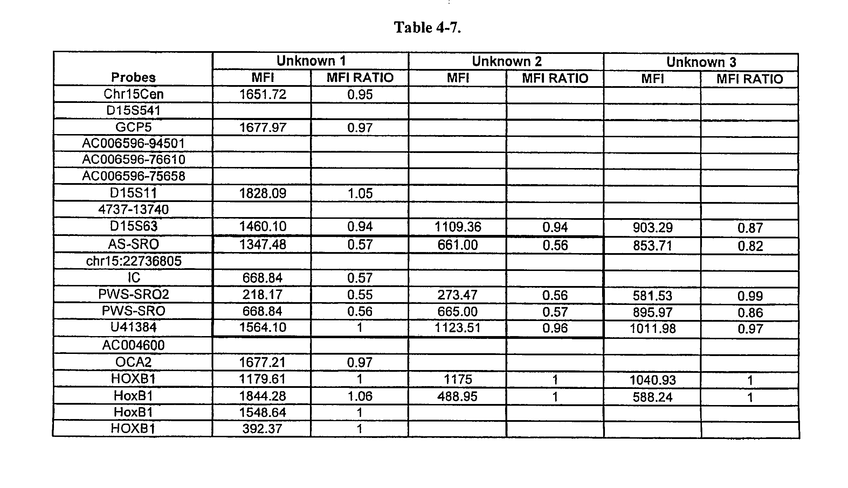

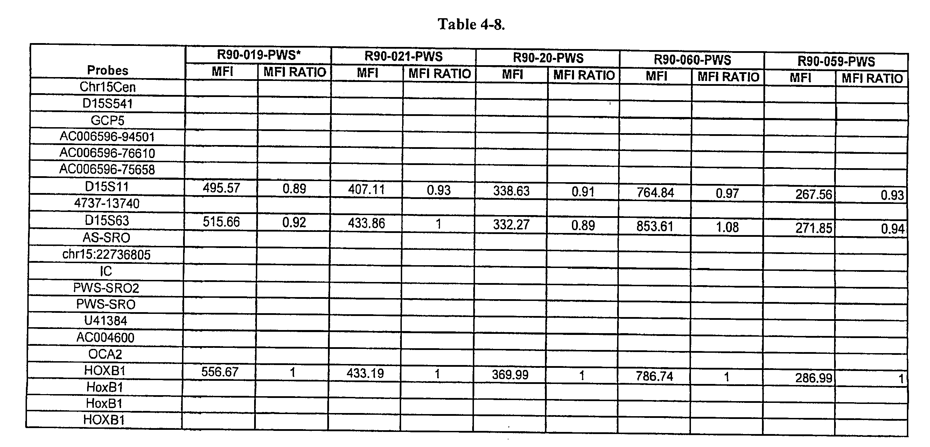

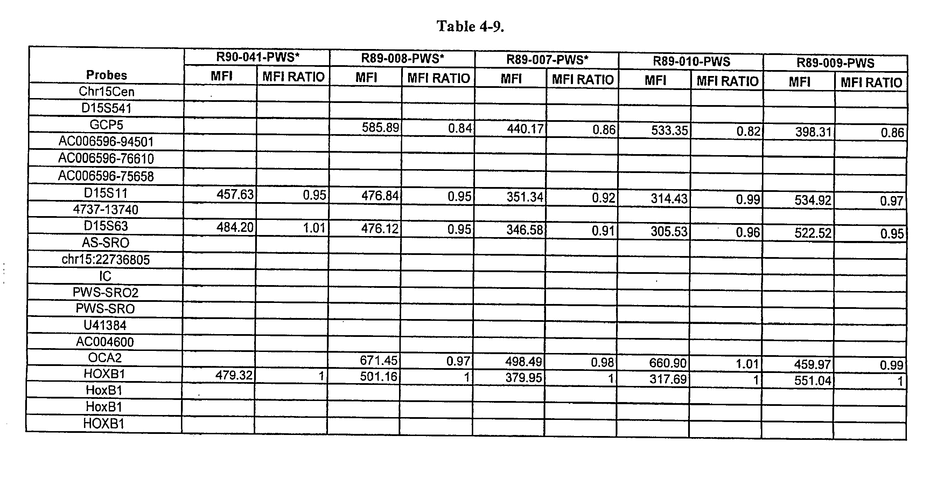

The genotypes of chromosome 9q34 deletion and normal control samples were distinguishable based on comparisons of fluorescence intensities. In a blind study, microspheres conjugated to 16-2b and HOXB1c were hybridized in separate reactions to Sample 33 and Sample 47 genomic DNA (Fig. 3). In the histograms of Fig. 3, the fluorescence intensity signal (FL2 phycoerythrin, x axis) versus the microsphere count (y-axis) is plotted for the test probe and the reference probe for each sample. The ratio of the mean fluorescence of the 16-2b probe to the *HOXB1c* reference probe (Table 2) indicates a lower copy number for 16-2b in Sample 33 (ratio = 0.67), consistent with a deletion of one copy of this locus, which was previously demonstrated by scFISH with probe 16-2a. Similar signal intensities were found for both probes in Sample 47 (ratio = 0.96), consistent with a normal genotype. The mean fluorescence ratios of probes 16-1b to HOXB1c in Samples 33 and 81 were also consistent with FISH results demonstrating a deletion of one copy of 16-1a in both patients.

Samples 33 and 38 were then hybridized with probes 16-1b, 16-2b, and HOXB1c in separate multiplexed reactions. Relative to the HOXB1c probe, Sample 33 showed mean fluorescence of 61% for 16-1b and 51% for 16-2b. Sample 38 hybridizations showed similar *HOXB1c* and 16-1b (87%) and 16-2b (89%) mean fluorescence levels. These results are consistent with the FISH data that demonstrate that Sample 33 has a hemizygous deletion for *ABL1* and Sample 38 does not.

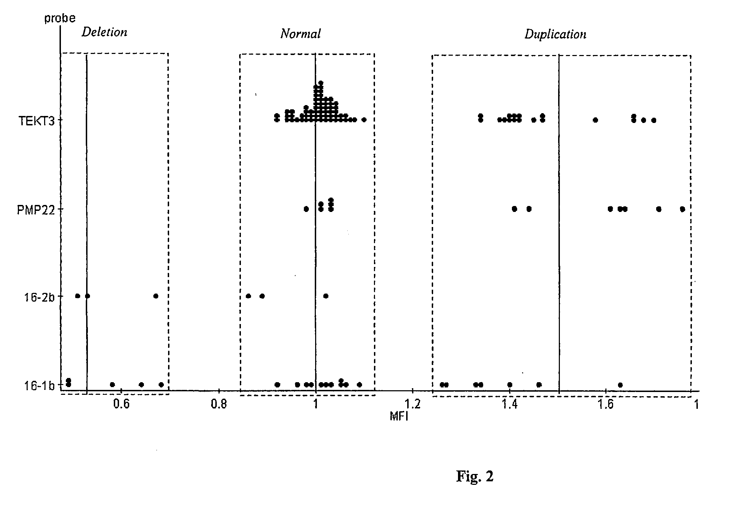

The ratio of the signal intensity of 16-1 to HOXB1 and 16-2 to HOXB1 for samples with *a* 5'*ABL1* deletion and for those without a deletion are graphically depicted in Fig. 2. In Fig. 2, samples are grouped by test probe (y-axis). A box (dashed lines) is drawn around each distinct genotypic group (left = chromosomal deletion, middle = normal, right = chromosomal duplication). Vertical lines indicate the theoretical MFI ratio for each genotype (0.5, 1.0, and 1.5 for deletion, normal and duplicated samples, respectively). As indicated, the MFI ratios for all samples within each genotype do not overlap with other genotypes. In general, all of the samples tested with a disomic complement have ratios for 16-1 or 16-2 derived probes relative to HOXB1 mean fluorescence intensities close to 1, and those samples with a 5'*ABL1* deletion have ratios close to 0.5 (Table 5). The geometric mean and median provide the most accurate measures of fluorescence intensity for signal in our hybridization assay, since these metrics have the smallest residual variance (0.10) in regression against the expected ratios for all of the genotypes studied.

### Detection of increased copy number of chromosome 9q34 in trisomic 9 cell lines

To determine iftriallelic copy number could be distinguished from biallelic copy number, To determine if triallelic copy number could be distinguished from biallelic copy number, probe 16-1b was hybridized to DNA from cell lines trisomic for 9q34 and compared with Sample 47, which is disomic for this region (Table 2). Both HOXB1c and 16-1b were multiplexed in a single hybridization reaction. Cell lines GM09286 (ratio = 1.34), GM10186 (ratio = 1.40) and GM06074 (ratio = 1.26) each exhibited significantly higher mean fluorescence for 16-1b compared to HOXB1c whereas both probes exhibited similar mean fluorescence signals for Sample 47 (ratio = 0.96). We suggest that a deviation from the expected ratio of 1.5 may be due to either the stability, i.e. low guanine-cytosine (GC) content, of 1c probe 16-1b, or to the secondary structure of the genomic region tested, resulting in fewer microsphere-bound targets. The assay confirms previous cytogenetic and FISH results showing the additional copy of the chromosome 9q34 sequence in all three cell lines and the normal copy number of the sequence in Sample 47 (Table 5).

### Detection of increased copy number of chromosome 17p12 region in CMT1A patients

Genomic DNA from five CMT1A patients with FISH-confirmed chromosome 17p12 duplications and Sample 86 with a normal karyotype in this chromosomal region were hybridized with either TEKT3 or PMP22 and HOXB1c 1c probes conjugated to microspheres. All CMT1A patient samples displayed similar elevations in mean fluorescence intensity signals (ranging from 28-40% greater than HOXB1c) for hybridization to TEKT3, reflecting the presence of three copies of this locus (Fig. 3). Sample 86 exhibited only a 2% difference in mean fluorescence signal intensities for the two probes (Table 2). Similar results were obtained for a multiplex analysis of the same CMT1A samples tested with *PMP22* and *HOXB1* (ranging from 28.5 to 54 % above HOXB1c; see Table 2). Independent multiplex hybridizations with microsphere-coupled TEKT3 and HOXB1 hybridized to CMT1A-1 and CMT1A-2 demonstrated that these results were reproducible, yielding mean fluorescence intensity ratios that varied by less than 2% for CMT1A-2 and by 16% for CMT1A-1 (Table 2). These ratios indicate a clustering around the expected value of 1.5 (1.26 - 1.85) for samples containing 17p12 duplications (Fig. 2) (Table 5).

The geometric mean or median fluorescence ratio was found to be the best measure for the hybridization assay because it provided the smallest confidence intervals and residuals for all of the genotypes with no overlap between genotypes (Fig. 2). Multiple linear regression of data for each ratio category against expected ratios over all genotypes gave correlation coefficients of 0.86 for the arithmetic mean, 0.89 for geometric mean and median, and 0.42 for the peak channel ratio. The 95% confidence intervals for the geometric mean fluorescence ratio of test to reference sample intensities for specimens with normal diploid genotype was 0.97 to 1.03, for hemizygous deletions was 0.5 to 0.64, and for duplications was from 1.44 to 1.56. The arithmetic mean produced slightly larger confidence intervals (residual variance = 0.13), and the peak channel fluorescence was found to be a suboptimal measure of copy number, because several hybridization reactions produced much larger residuals for these genotypes.

In a blinded study, triplicate reactions of 22 coded genomic samples (consisting of 20 normal and 2 CMT1A patient samples with previously confirmed duplications) were hybridized to TEKT3 and HOXB1c probes. Reproducibility for triplicate runs of each of these samples was excellent, with variances in the MFI ratios ranging from 6.5x10⁻⁶ to 1x10⁻³. Patient samples with CMT1A duplications were readily distinguishable from normal controls, based on the respective TEKT3:HOXB1c hybridization MFI ratios for these groups (summarized in Table 5). These are consistent with our previous results showing no overlap between these genotypes in our non-blinded analysis (Fig. 2).

### Distinguishing copy number genotypes

The MFI ratio is a robust metric for determining genomic copy number, regardless of which loci are analyzed. The average test to reference probe MFI ratio was 1.0±0.06 (n=21) in all normal individuals in the present study (Table 2). The ratio was 0.57±0.08 (n=8) for all samples with hemizygous deletions, and was 1.50± 0.15 (n=26), for triallelic samples. While several probes showed a modest degree of skewing of MFI ratios, abnormalities were clearly distinguishable from normal genotypes for all of the 1c probes at the 95% confidence level, and for the normal versus deleted genotypes, at the 98% confidence level.

### Discussion

Suspensions of spectrally-encoded polystyrene microspheres, coupled to synthetic DNA sequences, can be hybridized to genomic DNA and detected by conventional flow cytometry instruments. This approach has facilitated high throughput genotyping of single nucleotide polymorphisms (Vignali, 2000). However, prior art methodologies required that the target DNA be amplified prior to hybridizing the target DNA to a 10-50 nucleotide oligonucleotide probe coupled to microspheres (Hadd et al, 2004; Rockenbauer et al, 2005). Oligonucleotide probes cannot accurately quantify genomic copy number directly from patient DNA samples (Earley et al, 2002; Sekar et al, 2005; Vignali, 2000) because of inadequate sensitivity and specificity. Copy number estimation of target sequences is also complicated by the fact that the requisite amplification step can be difficult to control because it is inherently logarithmic. Genomic copy number can be unequivocally determined when directly-labeled genomic DNA is hybridized without prior amplification (Southern, 1975; White et al, 2004).

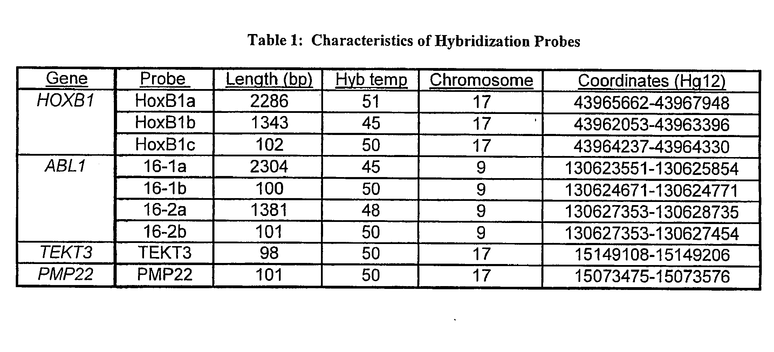

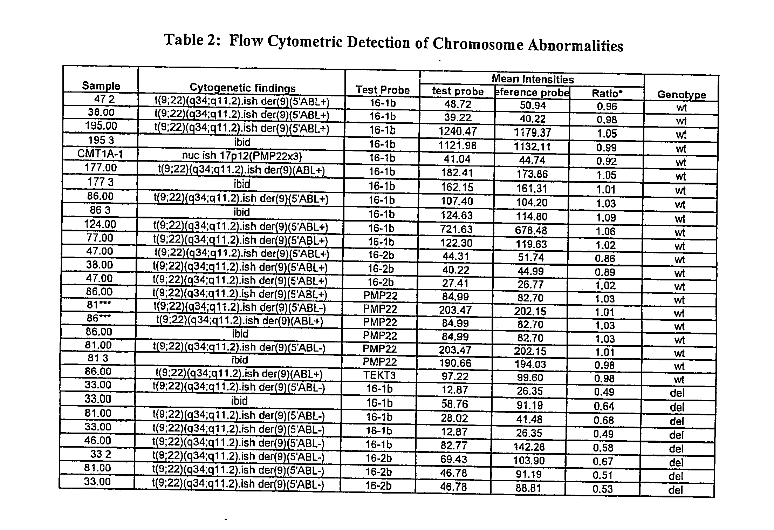

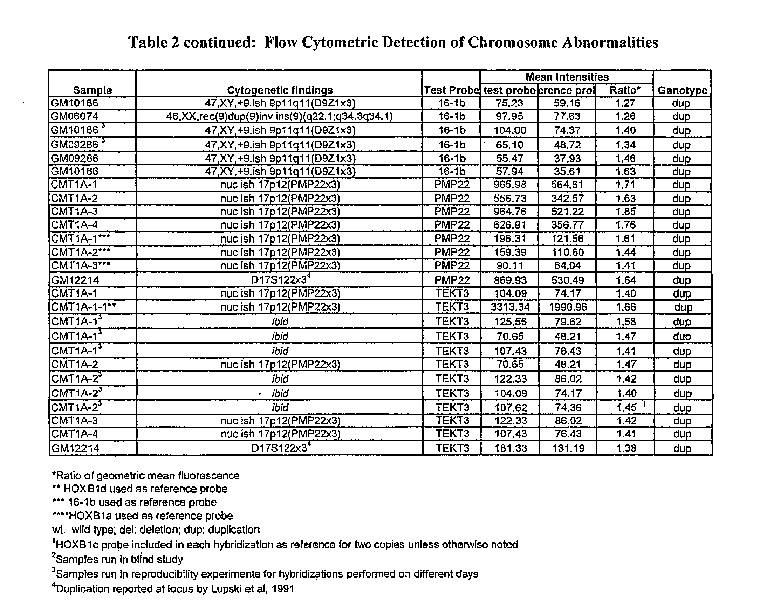

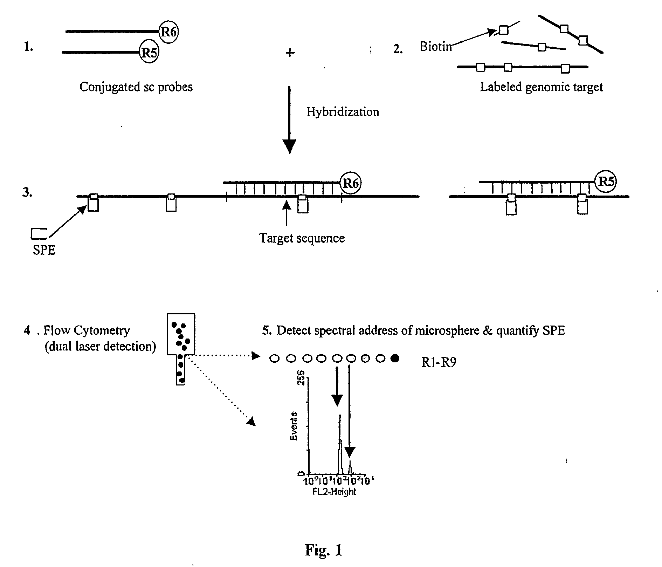

We examined the possibility that direct hybridization of patient-derived DNA to longer probes coupled to microspheres could quantitatively measure copy number (Fig. 1). In step 1) of this figure, each 1c probe is synthesized by PCR using an amino-modified forward primer and conjugated to spectrally distinct carboxylated microspheres. In step 2), sample DNA is extracted from fixed cytogenetic cell pellets and nick-translated to incorporate biotin dUTP. Step 3) illustrates that lc probe-coupled microspheres are hybridized to the prepared genomic DNA and stained with streptavidin-phycoerythrin (SPE) and washed to remove residual SPE. In step 4), samples are run on the flow cytometer (dual laser detection) for the detection of distinct spectral addresses of microspheres and quantification of target bound by each microsphere-coupled probe in step 5). Previously, we validated computationally-derived low copy (lc) probes to detect a wide variety of chromosomal abnormalities by fluorescence in situ hybridization (FISH) for many different chromosomal regions (Knoll and Rogan, 2003; Rogan et al, 2001). In this study, we developed microsphere suspension hybridization with lc probes from three different chromosomal regions associated with aneusomic conditions and demonstrated the use of this method to detect copy number gains or losses in archival, methanol-acetic acid fixed cytogenetic specimens.

With the present invention, a microsphere suspension hybridization assay to detect genomic copy number differences was developed which utilizes low copy (lc) genomic probes. Prior amplification of locus-specific targetDNA was not required since lc probes are designed to hybridize to a unique locus in the haploid genome sequence with high specificity. Loss or gain of low copy sequences can be directly detected in patient genomic DNA. This assay can follow routine cytogenetic analyses without requiring large patient sample quantities, additional blood draws, locus-specific genomic amplification or time-consuming genomic DNA purification methods. These studies were performed using fixed cell preparations remaining after cytogenetic analyses.

Hybridization experiments demonstrated adequate sensitivity to discern the presence of one versus two copies as well as two versus three copies of a genomic sequence. Use of multiple independent lc probes conjugated to microspheres with distinct spectral signatures can independently measure copy number changes in the same hybridization assay. Chromosome deletions were confirmed in the ABL1 gene in two CML patients, trisomy of chromosome 9q34 was confirmed in three cultured cell lines, and duplication of chromosome 17p12 was confirmed in cells of five CMT1A patients, including one cell line. The same probe used to detect a hemizyogous chromosome 9q34 deletion also detected three alleles in cell lines with trisomy at this locus. Ratios of probe mean or median fluorescence intensities were consistent for patients with the same genotype and were clustered around expected values, regardless of the chromosomal origin of the test probe.

Several parameters were optimized during assay development: (i) the lc probes conjugated to microspheres showed specificity for homologous sequences when examined in a heterologous complex genomic environment, i.e. as little as 5ng of target sequence present in 1 ug of heterologous genomic sequence was successfully detected; (ii) 1c probes conjugated to microsphere sets with different spectral addresses showed negligible differences in mean fluorescence intensities, regardless of which microsphere was conjugated to a probe; (iii) the length of the lc probe attached to the microsphere surface affected the efficiency of hybridization, with shorter probes (∼100 bp) exhibiting greater mean fluorescence intensities compared to longer probes (1 to 2 kb). The shorter probes were more stable (longer shelf life) resulting for less lot-to-lot variation in labeled microsphere stocks as well as (iv) this, in turn, reduced the effort required to conjugate, quantify and qualify DNA-bound microspheres.

### Example 2

This example describes a clinical application of quantitative microsphere hybridization to examine the Prader-Willi (PWS) and Angelman syndrome (AS) region on chromosome 15q11-13. 17 low copy and single copy test probes (80 nucleotides each) spanning ∼3.2Mb of the PWS/AS region and a disomic reference probe (HQXB1, chromosome 17q21), were each conjugated to one of ten spectrally distinct polystyrene microsphere levels. All probes were hybridized to biotin-labeled genomic patient DNA in multiplex QMH reactions, and hybridization was detected using phycoerythrin-labeled streptavidin and analyzed by dual-laser flow cytometry. Copy number differences were distinguished by comparing mean fluorescence intensities (MFI) of the test probes to the reference probe. The MFI ratios for deleted loci were 0.58±0.072 (n=80) as compared to the MFI ratios for normal loci, 0.98±0.075 (n=186).

Genomic rearrangements of chromosome 15q11-13 cause diverse phenotypes including autism, Prader-Willi syndrome (PWS), and Angelman syndrome (AS). This chromosomal region is subject to genomic imprinting and characterized by complex combinations of low copy repeat elements (refs). PWS and AS typically result from an ∼4Mb deletion in the paternal or maternal chromosome 15q11-13 region (Nichols 1998), respectively, with clustered breakpoints (BP) at either of two proximal sites (BP1 and BP2) and one distal site (BP3) which enables the classification of deletion patients (class I and II) (Knoll et al. 1990). Class I deletion patients are deleted for the region spanning BP1 to BP3 and class II deletion patients are deleted for the region spanning BP2 and BP3 (Knoll et al. 1990). Additionally, mutations within the bipartite imprinting center (IC) in this region can cause either disorder. One region of this IC is required for establishing and maintaining the paternal or maternal imprint, and studies have ascertained the shortest region of overlap (SRO) which defines the PWS-IC (PWS-SRO) and AS-IC (AS-SRO), (Mapendano et al. 2006). Further, both PWS and AS can arise from chromosome 15 uniparental disomy (UPD) which is maternal in origin in PWS patients (maternal disomy) or paternal in origin in AS patients (paternal disomy).

### Materials and Methods

*Probe selection, synthesis and microsphere conjugation.* A series of 17 different test lc probes specific to chromosome 15q11-13 were designed, as well as a disomic reference probe from HOXB1 on chromosome 17q21. Each of the probes was 80 bases in length as shown in Table 3. There was only one genomic match for the full 80 bases of each probe; however, small segments of probe may match other genomic regions.

Probes were selected based on their low copy sequence composition, GC content (48-55%), the lack of potential stable secondary sequence conformations as predicted by MFold software (available through The Bioinformatics Center at Rensselaer and Wadsworth, Rensselaer Polytechnic Institute, Troy, NY), and their length (80 nucleotides). Each probe was synthesized using a 5'-C6-amino-labeled forward primer (Integrated DNA Technologies, Coralville, IA) for coupling to carboxylated microspheres, and an unmodified reverse primer by PCR (Promega RedTaq Ready Mix, Madison, WI) using a normal human genomic control DNA (Promega) as template. PCR product was separated by gel electrophoresis (Seakem; FMC Bioproducts, Rockland, ME) and extracted by microspin column centrifugation (QiaQuik; Qiagen, Valencia, CA). Probes were conjugated to one of ten spectrally distinct microsphere levels (CytoPlex; Duke Scientific, Palo Alto, CA) via a carbodiimide coupling reaction (Dunbar et al. 2003; Fulton et al. 1997).

Each probe was initially heat denatured and then snap-cooled on ice. Approximately 3.125 x 105 microspheres with identical spectral characteristics were pipetted into a 1.5 mL microcentrifuge tube (USA Scientific, Ocala, Florida), centrifuged for 2 minutes at 10,000 g, and drained of supernatant. 150 µL of 0.1 M MES buffer (2-(N-morpholino)ethanesulfonic acid) pH4.5 was added to each tube and the microspheres were vortexed briefly followed by centrifugation for 2 minutes at 10,000 g. Supernatant was removed and the microspheres were resuspended by vortexing in 80 µL of 0.1M MES. A single lc probe (0.5 nmol) was added to each tube and mixed by vortexing. A 1.25 µL volume of fresh 10 mg/ml solution of 1-ethyl-3-3-dimethylaminopropyl carbodiimidehydrochloride (EDC) was added and the reaction was vortexed briefly and incubated in the dark for 30 minutes with occasional mixing. Mixing and incubation of EDC was repeated twice, using 1.25 µL of freshly prepared EDC solution each time. The reaction was stopped by addition of 500 µL 0.02% Tween20 followed by vortexing and centrifugation for 2 minutes at 10,000 g. Following removal of the supernatant, 250 µL of 0.1 % SDS was added to each tube, vortexed and then centrifuged at 10,000 g for 2 minutes. The supernatant was carefully removed, 25 µL of 0.1M MES pH4.5 was added and the tube was vortexed and stored in the dark at 4°C. Coupled microsphere concentrations were quantitated by adding 1 µL of each microsphere to 100uL of 1X PBS and analyzing on the FACSCalibur flow cytometer (Becton Dickinson, San Jose, California) using conditions given below.

Quality control procedures for each microsphere-conjugated probe were performed to ensure proper attachment of probes to microspheres. It was first determined that all probes could detect the corresponding PCR product in a hybridization reaction, establishing that probe conjugation and hybridization to microspheres were efficient. Purified PCR products specific to each of the QMH probe utilized in this study (Table 3) were amplified from normal genomic DNA (Promega) using a single 5' biotinylated primer. The biotinylated PCR products were hybridized to complementary microsphere-conjugated probes and detected with SPE. Hybridized microsphere count and mean fluorescence intensities were measured using CellQuest software (Becton Dickinson). The density of hybridized 1c products for a set of microspheres was estimated by comparing the quantity of single beads (from the gated side scatter bead count) to the number of hybridized beads (gated bead signal count and mean fluorescence intensity level), which determined the conjugation efficiency of the carbodiimide coupling procedure. The specificity of the microsphere-conjugated lc probe to its target sequence was determined by comparing mean fluorescence intensity of repeated flow cytometry runs of independent hybridization assays with homologous versus heterologous probes. Hybridization of each probe was optimized across a range of annealing temperatures (45°C to 60°C).

Additionally, microsphere-swap experiments were performed to verify that the MFI value was independent of the level of microsphere used for conjugation, the details for which were stated previously (Newkirk et al. 2006). Such experiments were performed using lc probe D15S63 conjugated to two microsphere sets with different spectral addresses (L2 and L9). Both microsphere sets were hybridized to corresponding PCR product in a multiplex reaction, and mean fluorescence intensity levels were compared for each microsphere set. Both reactions showed similar MFI values for the same probe. This experiment was repeated for lc probe IC (Table 3) with similar results.

*Patient samples and genomic target preparation.* Genomic DNA template was prepared using archived patient DNA samples (n=21) or using methanol-acetic acid fixed cell pellets derived from cytogenetic preparations of bone marrow samples (n=10). Fixed cell pellets from patients had been stored from two years to eight years in methanol and acetic acid. Genotypes for some samples were known from prior cytogenetic, microsatellite, and/or Southern hybridization studies, while some were unknown (Table 4-7). The fixed cells were washed twice with 1X PBS and their concentrations were determined with a hemocytometer. Genomic DNA template was extracted from ∼600 fixed cells per sample.

For archived DNA samples, no treatment prior to amplification was necessary. Whole genomic DNA was replicated *in vitro* using a modified protocol of the GenomiPhi kit (GE Healthcare, Piscataway, NJ) to allow for the direct incorporation of biotin into template DNA. First, 1 uL of each DNA sample was mixed with 9uL of Sample Buffer (GE Healthcare) and heat denatured for 3 minutes at 95°C. Samples were snap-cooled on ice for 2 minutes followed by addition of 4uL of 50nmol Biotin-16-dUTP (Roche, Indianapolis, IN), 9uL of Reaction Buffer (GE Healthcare), and 1uL of Enzyme Mix (GE Healthcare). Reactions were incubated at 30°C for 24-36 hours. Successful replication of patient DNA was verified by PCR amplification of the HOXB1 and PWS-SRO probes (Table 3). Any sample not yielding PCR product in this initial quality control step was not used for QMH. It was observed that samples that did not successfully amplify PCR product did not produce accurate QMH results (data not shown). The age of the fixed cell pellet is a likely contributing factor for the lack of PCR amplification following the modified GenomiPhi procedure. Analysis of these samples by PCR and gel electrophoresis indicated the extracted DNA was highly degraded and sheared, which would in turn cause an intact probe to appear deleted in QMH. It was observed that genomic DNA which passed the PCR quality control was obtained from more recently stored fixed cell pellets (∼2-5 years) versus older cell pellets (>6 years). DNA samples which passed the quality control PCR assay were sheared to ∼300-800bp by sonication (B-300, Bransonic, Danbury, CT), which was monitored by gel electrophoresis (Seakem). Twenty five ng of each labeled and fragmented genomic DNA sample was used per QMH reaction.

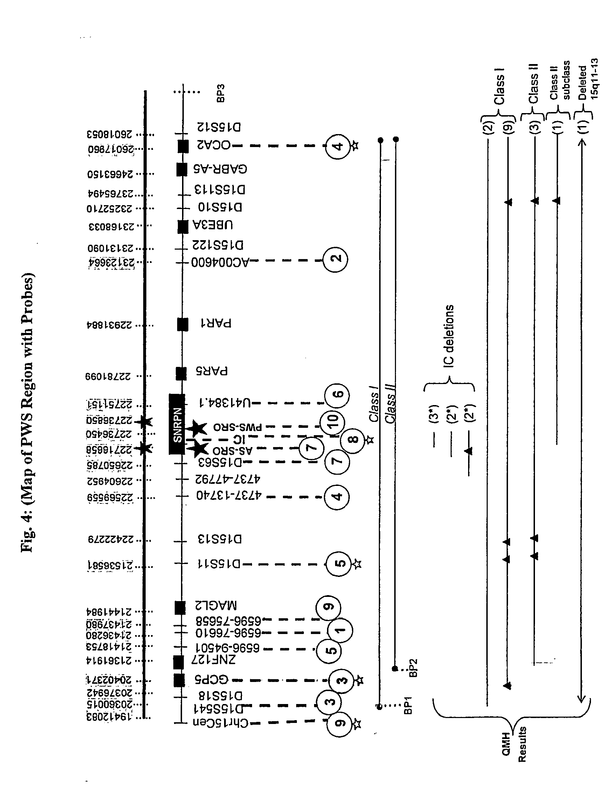

*Hybridization reactions and flow cytometry.* Prepared genomic DNA was diluted in 40uL of 1.5XTMAC hybridization buffer (3-mol/l tetramethylammonium chloride, 50mmol/l Tris-HCl, pH8.0, 1 g/l Sarkosyl) containing ∼7,500 of each probe-coupled microsphere. Multiplex analysis of two to ten different levels of microspheres conjugated to probes for QMH was possible in a single reaction using the CytoPlex microspheres (Duke Scientific; Palo Alto, CA). Probes included in hybridization reactions were selected based on available genotypic evidence in each previously characterized patient or family, and unknown samples were hybridized with chr15Cen, GCP5, D15S11, D15S63, PWS-SRO, and OCA2 initially in effort to delineate class I, class II, and IC deletions (Table 3). QMH probes used in subsequent hybridization reactions were selected adjacent to and within any deleted regions detected in the first QMH reaction in order to precisely characterize the deletion interval (Fig. 4). Reactions were heat denatured at 95°C for 5 minutes and allowed to hybridize overnight at 50°C. Reactions were centrifuged for 2 minutes at 10,000 g, the supernatant was removed, and 12 µL of a 1:50 dilution of a reporter molecule, streptavidin-phycoerythrin (SPE; Molecular Probes, Eugene, OR), in 1.5X TMAC was added to detect genomic targets containing biotin. The reactions were incubated at their hybridization temperature for 12 minutes. Following labeling, 250 µL of 1.5X TMAC was added to each reaction, mixed, and centrifuged at 10,000 g for 2 minutes. The supernatant was removed and the hybridized microspheres were resuspended in 70 µL 1.5X TMAC.

Samples were evaluated by flow cytometry analysis using a FACSCalibur (Becton Dickenson, San Jose, CA) and approximately 20,000 events were analyzed per reaction. Typical PMT voltage settings for the FACSCalibur instrument were FSC (forward scatter) = E00 (no signal amplification) in Log Mode, SSC (side scatter) = 344V in Linear Mode, FL1 = 628V in Logarithmic Mode, FL2 = 620V in Logarithmic Mode, FL3 = 710V in Logarithmic Mode, and FL4 = 500V in Logarithmic Mode. Thresholds for FL1, FL2, and FL3 were set at a default of 52V. The FSC threshold was the primary parameter with a value of 50V and the secondary parameter was SSC and set at 130V. The flow rate was set on low. Color compensation was set as follows: FL1=1.9% FL2, FL2=21 % FL1, FL2=0% FL3, FL3=10% FL2, FL3=4.7% FL4, and FL4=3.6% FL3. Color compensation values minimize the spectral overlap of MFI values measured in different channels of the flow cytometer. These settings were determined as optimal based on distinct clusters for each of the 10 different levels of unlabeled microspheres in the SSC vs bead signal plot. Fluorescent bead standards (LinearFlow Flow Cytometry Intensity Calibration Kit; Molecular Probes) were used for intensity calibration of different fluorochrome detection channels of the flow cytometer. The instrument was also calibrated with fluorescent reference standards (Quantum R-PE MESF Medium Level Kit; Bangs Laboratories, Fishers, Indiana), based on surface-labeled beads calibrated in molecules of equivalent soluble fluorochrome (MESF) units. After analysis of each sample by flow cytometry, the ratios of geometric mean fluorescence intensities (MFI) of test probe to reference probe were calculated and statistics (eg. mean, 95% confidence intervals, and standard deviation) were generated using MS Excel as shown in Tables 4-1 through 4-9 (referred to collectively as Table 4). Tables 4-1 through 4-9 each include probe identifiers in the leftmost column followed by sample results in paired columns including mean fluorescence intensities and mean fluorescence intensity ratios. Data showing deletions is bounded by a darkened boarder. Geometric MFI values have been previously shown to be the most accurate for assessing data from QMH assays since data is collected in logarithmic mode on the flow cytometer (Kirkwood 1979; Coder et al. 1994; Newkirk et al., 2006).

### Results

### Detection of Large Deletions in PWS/AS patients

A series of microsphere-conjugated lc probes spanning chromosome 15q11-q13 were hybridized to labeled genomic DNA from patients with large deletions in the PWS/AS region. In some instances deletions were determined by prior analysis (see Methods) (n=8; WJK18, 35, 29, 36, 48, C93-49-PWS, R92-161-PWS, R92-144-PWS), and in other patients the genotypes were unknown however a clinical diagnosis of PWS or AS had been made (n=3; Unkowns 1, 2 and 3).

A map of the PWS region with probes is provided in Fig. 4. In this figure, the chromosomal coordinates for lc probes conjugated to microspheres (highlighted) as well as other microsatellites and genes are indicated. The AS-SRO and PWS-SRO are identified with large stars. Probes conjugated to microspheres are indicated by a vertical dashed line adjoined to a circle representing the level of microsphere which was used for conjugation (1-10). The class I and class II deletion intervals as well as their breakpoint intervals (BP) are depicted. Small stars indicate which probe-conjugated microspheres were used in an initial QMH assay to distinguish class I from class II deletion patients. QMH results illustrate the deleted regions found in patients examined in the present study. Triangles placed on the deletion intervals mark the microsatellite markers tested in each group to define deletion intervals in previous studies (ref). The number of patients found with each type of deletion interval is indicated in parentheses and a black "*" indicates if these individuals are related.

Initial multiplex hybridization reactions to delineate class I from class II deletions in all large deletion patients (n=11) included test probes specific to Chr15Cen, GCP5, D15S11, PWS-SRO and OCA2, and the HOXB1 reference probe. The copy number for all probes was readily distinguishable based on MFI ratios in each patient (Tables 4-4, 4-5 and 4-6). The MFI ratio for deleted regions in this group of patients ranged from 0.44-0.67 and for intact genomic segments ranged from 0.83-1.06 (Tables 4-4, 4-5 and 4-6). Using the results from this initial QMH reaction, subsequent probes were chosen to further narrow each deletion interval. For example in patients WJK-29, WJK-36, and R92-144-PWS, Chr15Cen was intact but, GCP5, D15S11, PWS-SRO and OCA2 were found to be deleted (Tables 4-5 and 4-6). To further delineate these deletion intervals, a QMH probe specific to D15S541 between Chr15Cen and GCP5 was used in a second reaction. Results from this hybridization reaction revealed that WJK-29, WJK-36, and R92-144-PWS are also deleted for D15S541, which accounts for a ∼5.66Mb deletion interval, and classifies these two patients as class I (Amos-Landgraf et al. 1999). In patients WJK-18, 35, 48, and R92-161-PWS, initial QMH results indicated that Chr15cen and GCP5 were intact, but D15S11, PWS-SRO, and OCA2 were deleted (Tables 4-4 and 4-6). After a second QMH reaction, combined results showed a deletion from AC006596-94501 to OCA2 (∼4.6Mb), which is considered a class II deletion (Knoll et al. 1990).

QMH results for one patient revealed a non-classical deletion as compared to the typical deleted segment for class I and class II patients (Table 4). In patient C93-49-PWS, probes for D15S63, IC, U41384, AC004600, and OCA2 were present in one copy each, while Chr15Cen, GCP5, AC006596-94501, AC006596-76610, D15S11, and AC004737-13740 were disomic (Table 4-4). The deletion interval spans ∼3.36Mb and could represent a sub-class of class II deletions.

### Detection of IC Deletions in PWS patients

A total of six PWS patients previously found to have IC deletions by microsatellite analysis were used for QMH (R93-001-MOD, R93-002-MOD, R93-013-MOD, R90-035-PWS, R92-166-PWS, and R92-167-PWS) (Tables 4-1, 4-2 and 4-3). For the first family, test probes specific to AC006596-75658, AC004737-13740, D15S63, chr15:22736805, PWS-SRO, U41384, and AC004600, as well as the reference probe, HOXB1, were hybridized to labeled genomic DNA from each family member (Table 3). In R92-166-PWS and R92-167-PWS, a deletion from D15S63 to PWS-SRO was identified (∼93kb). Previous microsatellite results indicated a deletion at D15S63 and D15S128, so the QMH results extended the deletion found by microsatellite genotyping by ∼70kb. The unaffected family member (R92-168-PWS) was found normal for all tested loci (Table 4-1).

Another family with a known PWS IC mutation identified previously by Southern hybridization and microsatellite analysis was analyzed by QMH (R93-001-MOD, R93-002-MOD, R93-013-MOD, and R93-014-MOD) (Tables 4-2 and 4-3) (Ohta et al., 1999; Rogan and Knoll, 2003). Initial hybridization reactions included test probes for AC006596-94501, IC, PWS-SRO, and AC004600, in addition to the HOXB1 reference probe. Results showed that all samples analyzed were intact for AC006596-94501 and AC004600, but IC and PWS-SRO were deleted in R93-001-MOD, R93-002-MOD, and R93-013-MOD. Subsequent QMH reactions with test probes circumscribing this deletion interval refined the boundary of the IC deletion from AS-SRO to PWS-SRO (∼50kb), which extends previous results by ∼45kb (Buiting et al 1995; Ohta et al. 1999).

QMH was used to analyze the smallest PWS-SRO deletion interval documented to date (∼4.3kb), R90-035-PWS (Table 4-3) (Ohta et al. 1999). An initial hybridization included probes specific to D15S63, AS-SRO, IC, PWS-SRO, chr15:22736805, and U41384 in addition to the reference HOXB1 probe (Table 3). MFI ratios indicated all test probes were intact and thus another probe within the region separating the AS-SRO and PWS-SRO, PWS-SRO2, was designed and hybridized to this patient. The PWS-SRO2 probe is located 14.5kb 3' (telomeric) of the AS-SRO probe, and 11.7kb 5' (centromeric) of the original PWS-SRO QMH probe. An MFI ratio of 0.68 reveals this locus is present in one copy (Table 4-3). The PWS-SRO2 probe was also hybridized to other IC deletion patients in the current study (R92-166-PWS, R93-013-MOD, R93-002-MOD, and R93-001-MOD) and found deleted as expected with MFI ratios ranging from 0.51 to 0.67 (Tables 4-1 and 4-2).

A clinically diagnosed AS patient, Unknown 1, tested as normal by FISH analysis and normal for UPD, however an abnormal SNRPN methylation test was found (Table 4-7). Samples from Unknown 1's mother and father were identified as Unknown 2 and Unknown 3, respectively (Table 4-7). This combination of test results made this patient an ideal candidate for the new assay of the present invention. Using QMH, this unknown patient (Unknown 1), and parents of the patient (Unknown 2 and Unknown 3), were initially screened with Chr15Cen, GCP5, D15S11, PWS-SRO, and OCA2, and the HOXB1 reference probe. Results indicated that all probes were intact, except for PWS-SRO (Table 4). A second QMH analysis focused on the IC region with the AS-SRO and 1C probes. MFI ratios indicated that these probes were deleted in the patient and the mother, which constitutes a ∼19kb deletion around the AS-SRO region (Table 4). This result was confirmed by a third QMH hybridization with the PWS-SRO2 probe, circumscribed by the IC and PWS-SRO probe, and found deleted in both the patient and the mother (Table 4), as well as scFISH analysis with the AS-SRO probe.

### Detection of Proximal Deleted Chromosome 15q

A patient, B2002-00905, with a known proximal chromosome 15q deletion, B2002-00905, was initially characterized by routine cytogenetic analysis and subsequently analyzed using QMH. A multiplex QMH assay included test probes specific to Chr15Cen, D15S541, GCP5, D15S11, D15S63, AC004600, and OCA2 in addition to the reference probe. Results indicated all test probes deleted, accounting for a deletion spanning >6.6Mb, however no low copy probe sequences 5' (centromeric) of probe Chr15Cen could be identified due to heterochromatic sequence dense with repeats in this region, and thus the centromeric break in this inversion could not be refined.

### Evaluation of Asynchronous Replication Timing in Maternal Disomy PWS Patient Families

Previous studies (Knoll et al. 1990) examining allele specific replication (ASR) timing within chromosome 15q11-13 revealed paternal early/maternal late replication timing patterns at D15S11 and D15S63. Using QMH probes specific to these two loci, four PWS patients with chromosome 15 maternal disomy (R89-007-PWS, R89-008-PWS, R90-019-PWS, R90-041-PWS) and their family members were tested to see if ASR affected QMH ratios in affected individuals (n=10) (Tables 4-8 and 4-9). The HOXB1 reference probe was used in FISH hybridization studies to verify synchronous replication timing. QMH results for the maternal disomic individuals indicate that there is no discernable difference in the MFI ratios as compared to the normal parents. For instance, the MFI ratios for D15S11 and D15S63 in R90-041-PWS were 0.95 and 1.01 (Table 4-9), respectively, which is actually higher as compared to the father, R90-059-PWS, (0.93 and 0.94) but slightly less than the mother, R90-060-PWS, (0.97 and 1.08) (Table 4-8). QMH results for the other two families studied (R90-059-PWS, R90-041-PWS, R89-008-PWS, R89-010-PWS, R89-009-PWS) were synonymous with results for this family.

### Statistical Analysis of QMH Results for Normal and Deleted Loci

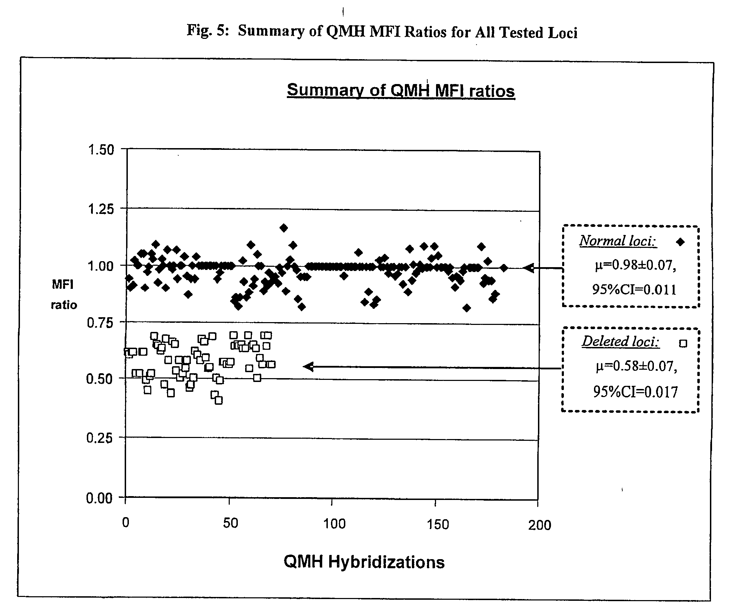

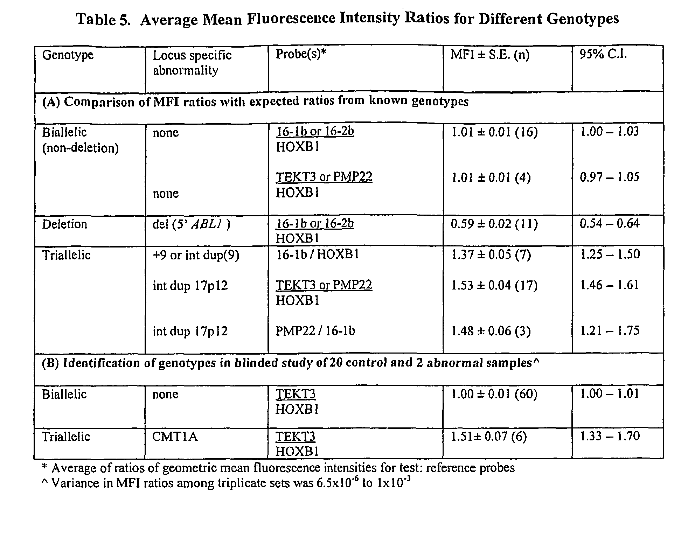

In Fig. 5, MFI ratios are plotted on the y-axis and the number of QMH hybridizations is plotted along the x-axis. There was no overlap in the MFI ratios for normal and deleted probes, hence the copy number at each locus was readily distinguishable. The mean, standard deviation, and 95% confidence intervals for normal and deleted loci are indicated in Fig. 5.

Analysis of the MFI values for all normal loci tested by QMH (n=191) showed a mean of 0.98±0.075 with a 95% confidence level of 0.011 (Fig. 5) and the MFI values for all deleted loci (n=80) have a mean of 0.58±0.072 with a 95% confidence level of 0.016 (Fig. 5). No overlap between the MFI values for normal and deleted loci (n=271) was observed and genotypes were easily distinguishable for all patients which passed the initial PCR quality control step (Fig. 5). It was observed that QMH assays for patient samples stored in methanol:acetic acid for a shorter length of time (∼2-5 years) prior to DNA extraction produced MFI ratios that were consistently closer to 1.0 and 0.5 for normal and deleted loci, respectively, as compared to those stored for longer lengths of time (>5 years). This is likely due to the compromised quality (ie. degraded and sheared condition) of the DNA obtained from fixed cell pellets which have been stored for excessive lengths of time. No patient sample stored for >6 years passed the initial PCR quality control step and thus could not be used for QMH analysis.

### Discussion

This study applies the previously developed quantitative microsphere hybridization (QMH) assay for the detection of copy number changes in the PWS/AS region on chromosome 15q11-13. Using QMH, class I and class II deletions were readily distinguished in a single assay and refined more precisely in a second QMH assay. Smaller deletions within the AS-SRO spanning ∼19kb were defined as well as deletions as small as 4.3kb within the PWS-SRO region. One patient exhibited an unusual deletion interval from D15S63 to OCA2 (∼3.36Mb), which could represent a new class or sub-class of deletions. Hybridization experiments illustrated adequate sensitivity to distinguish one vs. two copies of a genomic sequence for large and small deleted regions. QMH was unable, however, to discern asynchronous replication (ASR) timing patterns in PWS patients arising from maternal disomy. Future studies will include cell synchronization prior to target DNA extraction to further examine this potential application of QMH. The use of distinct spectrally encoded microspheres enabled accurate analysis of up to nine test probes and one reference probe in a single tube.

This study of the PWS/AS region is the first clinical application of the QMH assay and verifies that it is a suitable diagnostic alternative to other copy number determination methods not requiring context-dependent sequence identification. Other techniques to assess copy number in the same region include FISH, Southern hybridization, microarray, aCGH, MLPA, and MAPH. QMH reliably detected copy number differences at a genomic resolution of ≥ 80bp, which exceeds the resolution achievable by FISH, Southern hybridization, and aCGH (which is limited by the size and density of cloned probes, and is similar to MAPH and MLPA in probe density. QMH using lc probes does not require either target DNA amplification or the addition of competitor DNA (i.e. Cot-1 DNA) for the suppression of repetitive sequences. The addition of competitor DNA has been shown to compromise the accuracy and reproducibility of genomic hybridization assays (Newkirk et al. 2005), which would not be ideal for a clinical diagnostic test.

QMH was easily adaptable to the PWS/AS region, despite the high density of surrounding repetitive sequences and limited number of low copy regions available for probe design. Assay optimization was minimal (See Methods) and results were consistently reproducible between samples with similar genotypes with negligible differences between MFI ratios (Fig. 5; 0.98±0.075 for intact loci; 0.58±0.072 for deleted loci). With the exception of degraded genomic target DNA, no false positive or false negative results were obtained for samples analyzed in this study. This illustrates the requirement for quality control experiments prior to analysis by QMH to ensure accurate results. The QMH assay of the present invention is an ideal initial diagnostic test for the high-throughput screening of patients with PWS and AS. This test allows for the refinement of PWS/AS deletion intervals using subsequent QMH assays, and FISH could be used to confirm the chromosomal context of deletions detected by QMH.

The microsphere hybridization platform may be more appropriate in some diagnostic situations instead of FISH, array comparative genomic hybridization (aCGH), Southern analysis, multiplex amplifiable probe hybridization (MAPH), multiplex ligation probe hybridization (MLPA) and quantitative PCR (qPCR). The microsphere assay of the present invention improves resolution, increases signal to noise ratio, and is easily amenable to multiplexing, making it a high throughput approach. The ability to multiplex probes conserves available patient material and decreases time needed to make diagnoses. Further, contamination of heterologous genomic sequences was shown to not impact the hybridization efficiency in the assay.

In the QMH assay, differences in copy number are detected with a genomic resolution of as little as 100 bp at a level of less than 5 genomic equivalents per hybridization reaction. The level of resolution achieved with conventional FISH and Southern hybridization is ∼20 kb to 650 kb. With aCGH, the resolution is limited to the size and density of cloned probes and the variability in levels of fluorescence signal can affect accuracy of interpretation. MAPH has a resolution of approximately 50kb, and MLPA, while it has higher resolution than other techniques, is sensitive to single nucleotide polymorphisms at or near ligation sites which can lead to false negative results. Like qPCR, care must be taken to prevent contamination, and neither technique is easily multiplexed.

With genomic arrays of 1c microsphere-conjugated suspension hybridization probes, it should be feasible to rapidly delineate the extent of chromosomal abnormalities that alter normal copy number. Hybridization of a dense set of lc probes that span a chromosomal region could be used to initially screen for the boundaries of aneusomic domains. Subsequent FISH studies could then provide a chromosomal context for these genetic alterations. This invention lends itself to high throughput applications since, in theory, 3840 different products could be assayed on a single high density microtiter plate using our current set of 10 spectrally distinct encoded beads in each well. With current instrumentation, assuming a minimum probe resolution of 20kb, a single plate could provide high-resolution copy number determination for a 20Mb domain within 90 minutes. Based on the demonstrated sensitivity and accuracy of the method, microsphere suspension hybridization should be naturally extensible to other applications, such as the detection and quantification of low copy or rare mRNA sequences (∼5 genome equivalents per hybridization reaction) in cDNA.

### References for Example 1:

Armour JAL, Sismani C, Patsalis PC, Cross G. 2000. Measurement of locus copy number by hybridisation with amplifiable probes. Nucleic Acids Res. 28(2):605-609.
Bagwell CB, Baker D, Whetstone S, Munson M, Hitchcox S, Ault KA, Lovett EJ. 1989. A simple and rapid method for determining the linearity of a flow cytometer amplification system. Cytometry 10(6):689-94.
Brown RD, Zarbo RJ, Linden MD, Torres FX, Nakleh RE, Schultz D, Mackowiak PG. 1994. Two-color multiparametric method for flow cytometric DNA analysis. Standardization of spectral compensation. American Journal of Clinical Pathology 101(5):630-637.
Coder DM, Redelman D, Vogt RF. 1994. Computing the central location of immunofluorescence distributions: logarithmic data transformations are not always appropriate. Cytometry 18(2):75-8.
Dunbar S, Godbout R, Newkirk H, Hetzel J. 2003. Microsphere suspension array technology for SNP detection in cattle. IEEE Eng Med Biol Mag 22(4):158-62.
Earley M, Vogt RJ, Shapiro H, Mandy F, Kellar K, Bellisario R, Pass K, Marti G, Stewart C, Hannon W. 2002. Report from a workshop on multianalyte microsphere. Cytometry 50(5):239-42.
Fulton RJ, McDade RL, Smith PL, Kienker LJ, Kettman JR, Jr. 1997. Advanced multiplexed analysis with the FlowMetrix system. Clin Chem 43(9):1749-56.
Hadd AG, Laosinchai-Wolf W, Novak CR, Badgett MR, Isgur LA, Goldrick M, Walkerpeach CR. 2004. Microsphere bead arrays and sequence validation of 5/7/9T genotypes for multiplex screening of cystic fibrosis polymorphisms. J Mol Diagn 6(4):348-55.
Inoue K, Dewar K, Katsanis N, Reiter LT, Lander ES, Devon KL, Wyman DW, Lupski JR, Birren B. 2001. The 1.4-Mb CMT1A Duplication/HNPP Deletion Genomic Region Reveals Unique Genome Architectural Features and Provides Insights into the Recent Evolution of New Genes. Genome Research 11(6):1018-1033.
Knoll JH, Lichter P. 1994. In situ hybridization to metaphase chromosomes and interphase nuclei. In Dracopoli NC, Haines JL, Korf BR, Moir DT, Morton CC, Seidman CE, Seidman JG, Smith DR (eds): "Current protocols in Human Genetics Volume 1" Unit 4.3, Green-Wiley, New York. (Revised 2005, in press).
Knoll JH, Rogan PK. 2003. Sequence-based, in situ detection of chromosomal abnormalities at high resolution. American Journal of Medical Genetics 121(3):245-257.
Knoll JHM, Rogan PK. 2004. Single Copy Genomic Hybridization Probes and Method of Generating Same. US Patent No. 6,828,097.
Lewin B. 1980. DNA sequence organization: nonrepetitive and repetitive DNA. In "Gene Expression 2 - Eucaryotic Chromosomes", Chapter 18, pp 503-530, 2nd edition, Wiley-Interscience Publication.
Lupski JR, Oca-Luna RMd, Slaugenhaupt S, Pentao L, Guzzetta V, Trask BJ, Saucedo-Cardenas O, Barker DF, Killian JM, Garcia CA et al. 1991. DNA duplication associated with Charcot-Marie-Tooth disease type 1A. Cell 66(2):219-32.
Oostlander AE, Meijer GA, Ylstra B. 2004. Microarray-based comparative genomic hybridization and its applications in human genetics. Clin Genet 66(6):488-95.
Rockenbauer E, Petersen K, Vogel U, Bolund L, Kolvraa S, Nielsen KV, Nexo BA. 2005. SNP genotyping using microsphere-linked PNA and flow cytometric detection. Cytometry A 64(2):80-6.
Rogan PK, Cazcarro PM, Knoll JH. 2001. Sequence-based design of single-copy genomic DNA probes for fluorescence in situ hybridization. Genome Res 11(6):1086-94.
Schouten JP, McElgunn CJ, Waaijer R, Zwijnenburg D, Diepvens F, Pals G. 2002. Relative quantification of 40 nucleic acid sequences by multiplex ligation-dependent probe amplification. Nucleic Acids Res 30(12):e57.
Sekar M, Bloch W, John PS. 2005. Comparative study of sequence-dependent hybridization kinetics in solution and on microspheres. Nucleic Acids Res. 33(1):366-75.
Shaffer, LG, Bejjani, BA. 2004. A cytogeneticist's perspective on genomic microarrays. Hum Reprod Update. 10(3):221-26.
Sinclair P, Nacheva E, Leversha M, Telford N, Chang J, Reid A, Bench A, Champion K, Huntly B, Green A. 2000. Large deletions at the t(9;22) breakpoint are common and may identify a poor-prognosis subgroup of patients with chronic myeloid leukemia. Blood 95(3):738-43.
Southern EM. 1975. Detection of specific sequences among DNA fragments separated by gel electrophoresis. J Mol Biol 98:503-518.
Vignali D. 2000. Multiplexed particle-based flow cytometric assays. J Immunol Methods. 243(1-2):243-55.
White SJ, Breuning MH, den Dunnen JT. 2004. Detecting copy number changes in genomic DNA: MAPH and MLPA. Methods Cell Biol. 75:751-768.

### References for Example 2:

Amos-Landgraf JM, Ji Y, Gottlieb W, Depinet T, Wandstrat AE, Cassidy SB, Driscoll DJ, Rogan PK, Schwartz S, Nicholls RD (1999) Chromosome breakage in the Prader-Willi and Angelman syndromes involves recombination between large, transcribed repeats at proximal and distal breakpoints. Am J Hum Genet 65:370-386.
Bejjani BA, Shaffer LG (2004) A cytogeneticist's perspective on genomic microarrays. Hum Reprod Update 10:221-226
Bittel DC, Butler MG (2005) Prader-Willi syndrome: clinical genetics, cytogenetics and molecular biology. Expert Reviews in Molecular Medicine 7:1-20.
Bittel DC, KibiryevaN, Talebizadeh Z, Butler MG (2003) Microarray analysis of gene/transcript expression in Prader-Willi syndrome: deletion versus UPD. J Med Genet 40:568-574.
Bittel DC, Kibiryeva N, Talebizadeh Z, Driscoll DJ, Butler MG (2005) Microarray analysis of gene/transcript expression in Angelman syndrome: deletion versus UPD. Genomics 85:85-91.
Buiting K, Saitoh S, Gross S, Dittrich B, Schwartz S, Nicholls RD, Horsthemke B (1995) Inherited microdeletions in the Angelman and Prader-Willi syndromes define an imprinting centre on human chromosome 15. Nature Genetics 9:395-400
Knoll J, Rogan P (2004) Single Copy Genomic Hybridization Probes and Method of Generating Same.US 6,828,097
Knoll JH, Rogan PK (2003) Sequence-based, in situ detection of chromosomal abnormalities at high resolution. American Journal of Medical Genetics 121:245-257
Knoll JHM, Nicholls RD, Magenis RE, Glatt K, J.M. Graham J, Kaplan L, Lalande M (1990) Angelman syndrome: three molecular classes identified with chromosome 15q11q13-specific DNA markers. Am J Hum Genet 47:149-155
Mapendano CK, Kishino T, Miyazaki K, Kondo S, Yoshiura K-I, Hishikawa Y, Koji T, Niikawa N, Ohta T (2006) Expression of the Snurf-Snrpn IC transcript in the oocyte and its putative role in the imprinting establishment of the mouse 7C imprinting domain. J Hum Genet 51:236-243
Newkirk H, Knoll JHM, Rogan P (2005) Distortion of quantitative genomic and expression hybridization by Cot-1 DNA: mitigation of this effect. Nucleic Acids Research 33:e191
Newkirk H, Miralles M, Rogan P, Knoll JHM (2006) Determination of genomic copy number with quantitative microsphere hybridization. Human Mutation 27:376-386
Nygren AOH, AmezianeN, Duarte HMB, Vijzelaar RNCP, Waisfisz Q, Hess CJ, Schouten JP, Errami A (2005) Methylation-specific MLPA (MS-MLPA): simultaneous detection of CpG methylation and copy number changes of up to 40 sequences. Nucleic Acids Research 33:e128
Ohta T, Gray TA, Rogan PK, Buiting K, Gabriel JM, Saitoh S, Muralidhar B, Bilienska B, Krajewska-Walasek M, Driscoll DJ, Horsthemke B, Butler MG, Nicholls RD (1999) Imprinting-mutation mechanisms in Prader-Willi syndrome. Am J Hum Genet 64:397-413
Sahoo T, Shaw CA, Young AS, Whitehouse NL, Schroer RJ, Stevenson RE, Beaudet AL (2005) Array-based comparative genomic hybridization analysis of recurrent chromosome 15q rearrangements. Am J Med Genet 139A:106-113
Schouten JP, McElgunn CJ, Waaijer R, Zwijnenburg D, Diepvens F, Pals G (2002) Relative quantification of 40 nucleic acid sequences by multiplex ligation-dependent probe amplification. Nucleic Acids Res 30:e57

## Claims

1. A method of quantifying the copy number of a target nucleic acid sequence in a genome without preselection or amplification of the locus specific target sequence, which method includes a suspension hybridization assay, said method comprising the steps of:
extracting nucleic acid containing said target nucleic acid sequence from a subject, optionally replicating said nucleic acid in vitro,
attaching a label to said target nucleic acid including fragmenting the target nucleic acid,
preparing a particulate-conjugated, nucleic acid probe, specific for a low copy target sequence, of known sequence selected to complement said target nucleic acid sequence,
hybridizing said probe to said target nucleic acid to form a hybridization reaction product,
identifying said product through detection of said particulate, and
quantifying said copy number of said target nucleic acid sequence through detection of said label on said product,
wherein said low copy target sequence has a copy number of 10 or less.

2. The method of Claim 1, wherein said particulate comprises a nanocrystalline particle.

3. The method of Claim 1, wherein said particulate comprises a nanosphere.

4. The method of Claim 1, wherein said particulate comprises a microsphere.

5. The method of Claim 1, wherein said particulate comprises a spectrally-distinct polymer microsphere.

6. The method of Claim 2, wherein said microsphere comprises polystyrene.

7. The method of Claim 2, further comprising the step of binding a fluorochrome to a biological moiety on said microsphere surface.

8. The method of Claim 2, further comprising a step selected from the group consisting of conjugating said probes to said microsphere via modified carbodiimide reaction wherein said microsphere has been carboxylated and conjugating said microsphere to said probe using an electrophilic tether, wherein said tether comprises N-chloroacetamidohexyl phosphoramidite.

9. The method of Claim 1, wherein said particulate comprises an internally-dyed, fluorescent, polystyrene bead having a determined spectral address.

10. The method of Claim 1, wherein said extracted target nucleic acid comprises DNA selected from the group consisting of genomic DNA and complementary DNA, or RNA.

11. The method of Claim 1, wherein said target nucleic acid is labeled by a process selected from the group consisting of labeling via nick translation with an identifying label, directly labeled during an *in vitro* nucleic acid replication reaction, end labeling, and random priming.

12. The method of Claim 1, wherein said label is selected from the group consisting of fluorophores, enzymatic conjugates, fluorophore-tagged nucleotides, fluorescently-labeled antibodies bound to antigen-bearing nucleotides, biotin-dUTP, digoxygenin-dUTP, and combinations thereof.

13. The method of Claim 1, wherein the nucleic acid sequence of said probe is complementary to a single copy sequence in said subject's genome.

14. The method of Claim 1, wherein a target nucleic acid containing a deletion of one or more base pairs exhibits a response ratio of about 0.1 to about 0.75 in comparison to a reference probe having a normal complement of said target nucleic acid sequence.

15. The method of Claim 1, wherein a target nucleic acid containing an insertion or duplication of one or more base pairs exhibits a response ratio of about 1.25 to about 1.65 in comparison to a reference probe having a normal complement of said target nucleic acid sequence.

16. The method of Claim 1, wherein multiple probes having distinct nucleic acid sequences conjugated to particulates having one or more distinct spectral addresses are hybridized to target nucleic acids.

17. The method of Claim 1, wherein said particulate comprises a streptavidin-coated or carboxylated bead bound to a biotin moiety at the 3' or 5' terminus of said nucleic acid probe.

18. The method of Claim 1, wherein the amount of target sequence detected per 1 µg of heterologous genomic nucleic acid sequence is within the range of 50 pg to 5 ng.

19. The method of Claim 1, wherein differences in copy number are detected with a genomic resolution of as little as 60 bp.

20. The method of Claim 1, said quantifying step including the step of detecting the spectral address of said product using flow cytometry.

21. The method of Claim 1, said probe having a length of between 50 and 2500 bases.

22. The method of Claim 1, said particulate having a diameter between 50 nm and 1 µm.

23. A method of detecting a suspected chromosomal abnormality in subject-derived genomic or complementary nucleic acid, which method includes a suspension hybridization assay, said method comprising the steps of:
preparing a spectrally-encoded, fluorescent microsphere having a first spectral address,
identifying a genomic target nucleic acid probe sequence by ascertaining the nucleotide-by-nucleotide sequence of a target nucleic acid sequence wherein the abnormality is suspected to reside,
synthesizing a nucleic acid target probe, specific for a genomic low copy target sequence, of known sequence according to the identified genomic target nucleic acid probe sequence,
conjugating the genomic target probe to a microsphere having a first spectral address,
synthesizing a reference probe selected to hybridize to a reference nucleic acid sequence having a known copy number of said genomic target nucleic acid sequence,
conjugating the reference probe to a microsphere having a second spectral address,
reacting the target probe with a chromosomal target sequence containing the abnormality thereby causing the target probe to hybridize to the target sequence,
reacting the reference probe of known sequence with a chromosomal reference sequence containing said chromosomal target sequence and thereby causing the reference probe to hybridize to the reference sequence,
detecting the hybridized target probe to ascertain the existence of the chromosome abnormality,
detecting the hybridized reference probe, and
quantifying the detected hybridized target probe by comparing the response of the detected hybridized target probe with the response of the detected hybridized reference probe response,
without preselection or amplification of the locus specific target sequence, wherein said low-copy target sequence has a copy number of 10 or less.

24. The method of Claim 23, said abnormality being selected from the group consisting of differences in copy numbers of sequences within said genomic or complementary nucleic acid, duplications, deletions, inversions, transpositions, translocations, and combinations thereof.

25. The method of Claim 23, said method detecting said abnormalities with a genomic resolution of as little as 60 bp.

26. The method of claim 23, said detecting steps using flow cytometry.

27. A method of direct genomic quantitation of chromosomal abnormalities via flow cytometric detection of labeled genomic target nucleic acid sequences hybridized to microsphere-conjugated genomic or complementary nucleic acid probes, specific for a genomic low copy target sequence, of known sequence without preselection or amplification of the locus specific target sequence, said method comprising the steps of:
preparing a spectrally-encoded, fluorescent microsphere having a diameter between 50 nm and 1 µm,
synthesizing a target probe, specific for a genomic low copy target sequence, selected to hybridize to a particular nucleic acid sequence in the target wherein the abnormality is suspected to reside,
conjugating the target probe to the microsphere,
hybridizing the target probe to a target nucleic acid sequence,
labeling the target nucleic acid sequence,
detecting the hybridized target probe via flow cytometry, and
quantitating said abnormalities based on the results of said flow cytometry,
wherein the nucleic acid sequence of the probe is of a length of at least 60 bases, and wherein said low copy target sequence has a copy number of 10 or less.

28. The method of Claim 27, wherein the nucleic acid sequence of a probe is of a defined length between 60 and 2500 bases.

29. The method of Claim 27, said abnormality being selected from the group consisting of differences in copy numbers of sequences within said genomic or complementary nucleic acid, duplications, deletions, inversions, transpositions, translocations, and combinations thereof.

30. The method of Claim 27, said method detecting said abnormalities with a genomic resolution of as little as 60 bp.

31. A method of detecting chromosomal abnormalities without preselection or amplification of the locus specific target sequence, said method comprising the steps of:
preparing a hybridization probe of known sequence by coupling a spectrally-encoded, polystyrene microsphere to a synthetic DNA sequence complementary to a genomic low copy target sequence,
hybridizing said probe to genomic DNA,
labeling the genomic DNA,
detecting the product of said hybridization by flow cytometry,
wherein the nucleic acid sequence of the probe is of a length of at least 60 bases, and wherein said low copy target sequence has a copy number of 10 or less.

32. The method of Claim 31, said abnormality being selected from the group consisting of differences in copy numbers of sequences within said genomic or complementary nucleic acid, duplications, deletions, inversions, transpositions, translocations, and combinations thereof.

33. The method of Claim 31, said method detecting said abnormalities with a genomic resolution of as little as 60 bp.

34. The method of claim 31, further comprising the step of comparing the results from said flow cytometry with flow cytometry results from a hybridization product having a known copy number corresponding to said abnormality.

35. The method of Claim 31, wherein said probe is complementary to 10 or fewer locations in the genomic DNA.

36. The method of Claim 31, said DNA being unamplified.

37. The method of Claim 1, wherein said probe is of known sequence prior to hybridization.

38. The method of Claim 1, wherein the conditions under which hybridization occurs are tailored to the particular sequence of the probe.

39. The method of Claim 38, wherein the melting point of the probe is known prior to hybridization.

40. The method of Claim 1, wherein said genome is the human genome.

41. The method of Claim 1, wherein the G-C content of the probe is known prior to hybridization.

42. The method of Claim 1, wherein there is specific hybridization to said target nucleic acid sequence.

43. The method of Claim 1, wherein the sequence of the target nucleic is known prior to hybridization.

44. The method of Claim 1, further comprising a three-dimensional suspension array.

45. The method of Claim 1, wherein said hybridization of said probe is the result of a hybridization reaction.

## Patentansprüche

1. Verfahren zur Quantifizierung der Kopienzahl einer Zielnukleinsäuresequenz in einem Genom ohne Vorauswahl oder Amplifikation der Locus-spezifischen Zielsequenz, wobei das Verfahren einen Suspensionshybridisierungsassay umfasst, wobei das Verfahren die folgenden Schritte umfasst:
Extrahieren von Nukleinsäure, die die Zielnukleinsäuresequenz enthält, aus einem Versuchsobjekt, gegebenenfalls Replizieren der Nukleinsäure in vitro,
Anbringen einer Markierung an der Zielnukleinsäure, was die Fragmentierung der Zielnukleinsäure umfasst,
Herstellen einer Partikel-konjugierten Nukleinsäuresonde, die für eine Zielsequenz mit niedriger Kopienzahl spezifisch ist, aus einer bekannten Sequenz, die zum Komplementieren der Zielnukleinsäuresequenz ausgewählt ist,
Hybridisieren der Sonde an die Zielnukleinsäure unter Bildung eines Hybridisierungsreaktionsproduktes,
Identifizieren des Produktes durch Detektion des Partikels und
Quantifizieren der Kopienzahl der Zielnukleinsäuresequenz durch Detektion der Markierung an dem Produkt,
wobei die Zielsequenz mit niedriger Kopienzahl eine Kopienzahl von 10 oder weniger aufweist.

2. Verfahren nach Anspruch 1, wobei das Partikel ein nanokristallines Partikel umfasst.

3. Verfahren nach Anspruch 1, wobei das Partikel ein Nanokügelchen umfasst.

4. Verfahren nach Anspruch 1, wobei das Partikel Mikrokügelchen umfasst.

5. Verfahren nach Anspruch 1, wobei das Partikel ein spektral charakteristisches Polymermikrokügelchen umfasst.

6. Verfahren nach Anspruch 2, wobei das Mikrokügelchen Polystyrol umfasst.

7. Verfahren nach Anspruch 2, ferner umfassend den Schritt des Bindens eines Fluoreszenzfarbstoffes an eine biologische Komponente auf der Oberfläche des Mikrokügelchens.

8. Verfahren nach Anspruch 2, ferner umfassend einen Schritt, ausgewählt aus der Gruppe, bestehend aus Konjugieren der Sonden an das Mikrokügelchen mittels einer modifizierten Carbodiimidreaktion, wobei das Mikrokügelchen carboxyliert wurde, und Konjugieren des Mikrokügelchens an die Sonde unter Verwendung einer elektrophilen Kette, wobei die Kette N-Chloracetamidohexylphosphoramidit umfasst.

9. Verfahren nach Anspruch 1, wobei das Partikel ein innen gefärbtes, fluoreszierendes Polystyrolkügelchen mit einer bestimmten Spektraladresse umfasst.

10. Verfahren nach Anspruch 1, wobei die extrahierte Zielnukleinsäure DNA, ausgewählt aus der Gruppe, bestehend aus genomischer DNA und komplementärer DNA, oder RNA umfasst.

11. Verfahren nach Anspruch 1, wobei die Zielnukleinsäure durch ein Verfahren, ausgewählt aus der Gruppe, bestehend aus Markieren mittels Nick-Translation mit einer Identifizierungsmarkierung, die direkt während einer In-vitro-Nukleinsäurereplikationsreaktion markiert wird, End-Markierung und Oligolabelling-Technik, markiert wird.

12. Verfahren nach Anspruch 1, wobei die Markierung ausgewählt ist aus der Gruppe, bestehend aus Fluorophoren, enzymatischen Konjugaten, Fluorophor-markierten Nukleotiden, fluoreszierend markierten Antikörpern, die an Antigen-tragende Nukleotide gebunden sind, Biotin-dUTP, Digoxygenin-dUTP und Kombinationen davon.

13. Verfahren nach Anspruch 1, wobei die Nukleinsäuresequenz der Sonde komplementär zu einer Einzelkopiesequenz im Genom des Versuchsobjektes ist.

14. Verfahren nach Anspruch 1, wobei eine Zielnukleinsäure, enthaltend eine Deletion von einem oder mehreren Basenpaaren, im Vergleich zu einer Referenzsonde mit einem normalen Komplement der Zielnukleinsäuresequenz ein Reaktionsverhältnis von etwa 0,1 bis etwa 0,75 aufweist.

15. Verfahren nach Anspruch 1, wobei eine Zielnukleinsäure, enthaltend eine Insertion oder Duplikation von einem oder mehreren Basenpaaren, im Vergleich zu einer Referenzsonde mit einem normalen Komplement der Zielnukleinsäuresequenz ein Reaktionsverhältnis von etwa 1,25 bis etwa 1,65 aufweist.

16. Verfahren nach Anspruch 1, wobei mehrere Sonden mit unterschiedlichen Nukleinsäuresequenzen, konjugiert an Partikel mit einer oder mehreren unterschiedlichen Spektraladressen, an Zielnukleinsäuren hybridisiert werden.

17. Verfahren nach Anspruch 1, wobei das Partikel ein Streptavidin-beschichtetes oder carboxyliertes Kügelchen, gebunden an eine Biotinkomponente am 3'- oder 5'-Terminus der Nukleinsäuresonde, umfasst.

18. Verfahren nach Anspruch 1, wobei die Menge an Zielsequenz, die pro 1 µg heterologer genomischer Nukleinsäuresequenz detektiert wird, im Bereich von 50 pg bis 5 ng liegt.

19. Verfahren nach Anspruch 1, wobei Differenzen in der Kopienzahl mit einer genomischen Auflösung von nur 60 bp detektiert werden.

20. Verfahren nach Anspruch 1, wobei der Quantifizierungsschritt den Schritt der Detektion der Spektraladresse des Produktes unter Verwendung von Durchflusszytometrie umfasst.

21. Verfahren nach Anspruch 1, wobei die Sonde eine Länge zwischen 50 und 2500 Basen hat.

22. Verfahren nach Anspruch 1, wobei das Partikel einen Durchmesser zwischen 50 nm und 1 µm hat.

23. Verfahren zur Detektion einer mutmaßlichen Chromosomenanomalie in genomischer oder komplementärer Nukleinsäure von einem Versuchsobjekt, wobei das Verfahren einen Suspensionshybridisierungsassay umfasst, wobei das Verfahren die Schritte umfasst:
Herstellen eines spektral-codierten, fluoreszierenden Mikrokügelchens mit einer ersten Spektraladresse,
Identifizieren einer genomischen Zielnukleinsäuresondensequenz, indem Nukleotid für Nukleotid die Sequenz einer Zielnukleinsäuresequenz, in der sich die Anomalie vermutlich befindet, ermittelt wird,
Synthetisieren einer Nukleinsäurezielsonde, die für eine genomische Zielsequenz mit niedriger Kopienzahl spezifisch ist, aus einer bekannten Sequenz gemäß der identifizierten genomischen Zielnukleinsäuresondensequenz,
Konjugieren der genomischen Zielsonde an ein Mikrokügelchen mit einer ersten Spektraladresse,
Synthetisieren einer Referenzsonde, die für die Hybridisierung an eine Referenznukleinsäuresequenz mit einer bekannten Kopienzahl der genomischen Zielnukleinsäuresequenz ausgewählt ist,
Konjugieren der Referenzsonde an ein Mikrokügelchen mit einer zweiten Spektraladresse,
Reagierenlassen der Zielsonde mit einer Chromosomenzielsequenz, die die Anomalie enthält, wodurch erreicht wird, dass die Zielsonde an die Zielsequenz hybridisiert,
Reagierenlassen der Referenzsonde der bekannten Sequenz mit einer Chromosomenreferenzsequenz, welche die Chromosomenzielsequenz enthält, wodurch erreicht wird, dass die Referenzsonde an die Referenzsequenz hybridisiert,
Detektieren der hybridisierten Zielsonde zum Ermitteln der Existenz der Chromosomenanomalie,
Detektieren der hybridisierten Referenzsonde, und
Quantifizieren der detektierten Zielsonde durch Vergleichen der Reaktion der detektierten hybridisierten Zielsonde mit der Reaktion der detektierten hybridisierten Referenzsonde,
ohne Vorauswahl oder Amplifikation der Locus-spezifischen Zielsequenz,
wobei die Zielsequenz mit niedriger Kopienzahl eine Kopienzahl von 10 oder weniger aufweist.

24. Verfahren nach Anspruch 23, wobei die Anomalie ausgewählt ist aus der Gruppe bestehend aus Differenzen in den Kopienzahlen von Sequenzen innerhalb der genomischen oder komplementären Nukleinsäure, Duplikationen, Deletionen, Inversionen, Transpositionen, Translokationen und Kombinationen davon.

25. Verfahren nach Anspruch 23, wobei das Verfahren die Anomalien mit einer genomischen Auflösung von nur 60 bp detektiert.

26. Verfahren nach Anspruch 23, wobei die Detektionsschritte Durchflusszytometrie nutzen.

27. Verfahren zur direkten genomischen Quantifizierung von Chromosomenanomalien mittels Durchflusszytometriedetektion markierter genomischer Zielnukleinsäuresequenzen, hybridisiert an Mikrokügelchen-konjugierte genomische oder komplementäre Nukleinsäuresonden, die für eine genomische Zielsequenz mit niedriger Kopienzahl spezifisch sind, einer bekannten Sequenz ohne Vorauswahl oder Amplifikation der Locus-spezifischen Zielsequenz, wobei das Verfahren die Schritte umfasst:
Herstellen eines spektral-codierten, fluoreszierenden Mikrokügelchens mit einem Durchmesser zwischen 50 nm und 1 µm,
Synthetisieren einer Zielsonde, die für eine genomische Zielsequenz mit einer niedrigen Kopienzahl spezifisch ist, ausgewählt für die Hybridisierung an eine bestimmte Nukleinsäuresequenz in dem Ziel, in dem die Anomalie vermutet wird,
Konjugieren der Zielsonde an das Mikrokügelchen,
Hybridisieren der Zielsonde an die Zielnukleinsäuresequenz,
Markieren der Zielnukleinsäuresequenz,
Detektieren der hybridisierten Zielsonde mittels Durchflusszytometrie, und Quantifizieren der Anomalien basierend auf den Ergebnissen der Durchflusszytometrie,
wobei die Nukleinsäuresequenz der Sonde eine Länge von mindestens 60 Basen hat, und
wobei die Zielsequenz mit niedriger Kopienzahl eine Kopienzahl von 10 oder weniger aufweist.

28. Verfahren nach Anspruch 27, wobei die Nukleinsäuresequenz einer Sonde eine definierte Länge zwischen 60 und 2500 Basen hat.

29. Verfahren nach Anspruch 27, wobei die Anomalie ausgewählt ist aus der Gruppe, bestehend aus Differenzen in den Kopienzahlen der Sequenzen innerhalb der genomischen oder komplementären Nukleinsäure, Duplikationen, Deletionen, Inversionen, Transpositionen, Translokationen und Kombinationen davon.

30. Verfahren nach Anspruch 27, wobei das Verfahren die Anomalien mit einer genomischen Auflösung von nur 60 bp detektiert.

31. Verfahren zum Detektieren von Chromosomenanomalien ohne Vorauswahl oder Amplifikation der Locus-spezifischen Zielsequenz, wobei das Verfahren die Schritte umfasst:
Herstellung einer Hybridisierungssonde bekannter Sequenz durch Koppeln eines spektral-codierten Polystyrolmikrokügelchens an eine synthetische DNA-Sequenz, komplementär zu einer genomischen Zielsequenz mit niedriger Kopienzahl,
Hybridisieren der Sonde an genomische DNA,
Markieren der genomischen DNA,
Detektieren des Produktes der Hybridisierung mittels Durchflusszytometrie,
wobei die Nukleinsäuresequenz der Sonde eine Länge von mindestens 60 Basen hat, und
wobei die Zielsequenz mit niedriger Kopienzahl eine Kopienzahl von 10 oder weniger aufweist.

32. Verfahren nach Anspruch 31, wobei die Anomalie ausgewählt ist aus der Gruppe, bestehend aus Differenzen in den Kopienzahlen von Sequenzen innerhalb der genomischen oder komplementären Nukleinsäure, Duplikationen, Deletionen, Inversionen, Transpositionen, Translokationen und Kombinationen davon.

33. Verfahren nach Anspruch 31, wobei das Verfahren die Anomalien mit einer genomischen Auflösung von nur 60 bp detektiert.

34. Verfahren nach Anspruch 31, ferner umfassend den Schritt des Vergleichens der Ergebnisse aus der Durchflusszytometrie mit den Ergebnissen der Durchflusszytometrie von einem Hybridisierungsprodukt mit einer bekannten Kopienzahl entsprechend der Anomalie.

35. Verfahren nach Anspruch 31, wobei die Sonde komplementär zu 10 oder weniger Stellen in der genomischen DNA ist.

36. Verfahren nach Anspruch 31, wobei die DNA nicht amplifiziert ist.

37. Verfahren nach Anspruch 1, wobei die Sonde vor der Hybridisierung eine bekannte Sequenz hat.

38. Verfahren nach Anspruch 1, wobei die Bedingungen, unter denen die Hybridisierung stattfindet, an die jeweilige Sequenz der Sonde angepasst werden.

39. Verfahren nach Anspruch 38, wobei der Schmelzpunkt der Sonde vor der Hybridisierung bekannt ist.

40. Verfahren nach Anspruch 1, wobei das Genom das menschliche Genom ist.

41. Verfahren nach Anspruch 1, wobei der GC-Gehalt der Sonde vor der Hybridisierung bekannt ist.

42. Verfahren nach Anspruch 1, wobei eine spezifische Hybridisierung an die Zielnukleinsäuresequenz erfolgt.

43. Verfahren nach Anspruch 1, wobei die Sequenz der Zielnukleinsäure vor der Hybridisierung bekannt ist.

44. Verfahren nach Anspruch 1, ferner umfassend eine dreidimensionale Suspensionsanordnung.

45. Verfahren nach Anspruch 1, wobei die Hybridisierung der Sonde das Ergebnis einer Hybridisierungsreaktion ist.

## Revendications

1. Procédé de quantification du nombre de copies d'une séquence d'acide nucléique cible dans un génome sans présélection ou amplification de la séquence cible spécifique de locus, lequel procédé comprend un dosage d'hybridation de suspension, ledit procédé comprenant les étapes de :
l'extraction de l'acide nucléique contenant ladite séquence d'acide nucléique cible d'un sujet, réplication optionnelle dudit acide nucléique in vitro,
l'attache d'un marqueur audit acide nucléique cible comprenant la fragmentation de l'acide nucléique cible,
la préparation d'une sonde d'acide nucléique conjuguée à de la matière particulaire, spécifique pour une séquence cible à faible nombre de copies, de séquence connue sélectionnée pour compléter ladite séquence d'acide nucléique cible,
l'hybridation de ladite sonde audit acide nucléique cible pour former un produit de réaction d'hybridation,
l'identification dudit produit par détection de ladite matière particulaire, et
la quantification dudit nombre de copies de ladite séquence d'acide nucléique par détection dudit marqueur sur ledit produit,
dans lequel ladite séquence cible à faible nombre de copies a un nombre de copies de 10 ou moins.

2. Procédé de la revendication 1, dans lequel ladite matièree particulaire comprend une particule nanocristalline.

3. Procédé de la revendication 1, dans lequel ladite matière particulaire comprend une nanosphère.

4. Procédé de la revendication 1, dans lequel ladite matière particulaire comprend une microsphère.

5. Procédé de la revendication 1, dans lequel ladite matière particulaire comprend une microsphère polymère spectralement distincte.

6. Procédé de la revendication 2, dans lequel ladite microsphère comprend du polystyrène.

7. Procédé de la revendication 2, comprenant en outre l'étape de liaison d'un fluorochrome à un groupe caractéristique biologique sur ladite surface de microsphère.

8. Procédé de la revendication 2, comprenant en outre une étape sélectionnée dans le groupe consistant à conjuguer lesdites sondes à ladite microsphère par une réaction avec du carbodiimide modifié dans lequel ladite microsphère a été carboxylée et à conjuguer ladite microsphère à ladite sonde en utilisant une attache électrophile, ladite attache comprenant du N-chloroacétamidohexyl phosphoramidite.

9. Procédé de la revendication 1, dans lequel ladite matière particulaire comprend une bille de polystyrène fluorescent intérieurement teintée ayant une adresse spectrale déterminée.

10. Procédé de la revendication 1, dans lequel ledit acide nucléique cible extrait comprend de l'ADN sélectionné dans le groupe consistant en ADN génomique et ADN complémentaire ou ARN.

11. Procédé de la revendication 1, dans lequel ledit acide nucléique cible est marqué par un processus sélectionné dans le groupe consistant en marquage par translation de coupure avec un marqueur d'identification, marquage direct pendant une réaction de réplication d'acide nucléique *in vitro,* marquage des extrémités et amorçage aléatoire.

12. Procédé de la revendication 1, dans lequel ledit marqueur est sélectionné dans le groupe consistant en fluorophores, conjugués enzymatiques, nucléotides marqués au fluorophore, anticorps à marquage fluorescent liés aux nucléotides portant des antigènes, biotine-dTUP, dioxygénine-dUTP et combinaisons de ceux-ci.

13. Procédé de la revendication 1, dans lequel la séquence d'acide nucléique de ladite sonde est complémentaire d'une séquence à copie unique dans ledit génome du sujet.

14. Procédé de la revendication 1, dans lequel un acide nucléique cible contenant une délétion d'une ou plusieurs paires de bases présente un taux de réponse d'environ 0,1 à environ 0,75 par comparaison avec une sonde de référence ayant un complément normal de ladite séquence d'acide nucléique cible.

15. Procédé de la revendication 1, dans lequel un acide nucléique cible contenant une insertion ou duplication d'une ou plusieurs paires de bases présente un taux de réponse d'environ 1,25 à environ 1,65 par comparaison avec une sonde de référence ayant un complément normal de ladite séquence d'acide nucléique cible.

16. Procédé de la revendication 1, dans lequel de multiples sondes ayant des séquences d'acide nucléique distinctes conjuguées à des matières particulaires ayant une ou plusieurs adresses spectrales distinctes sont hybridées à des acides nucléiques cibles.

17. Procédé de la revendication 1, dans lequel ladite matière particulaire comprend une bille revêtue de streptavidine ou carboxylée liée à un groupe caractéristique biotine à la terminaison 3' ou 5' de ladite sonde d'acide nucléique.

18. Procédé de la revendication 1, dans lequel la quantité de séquence cible détectée par 1 µg de séquence d'acide nucléique génomique hétérologue est de l'ordre de 50 pg à 5 ng.

19. Procédé de la revendication 1, dans lequel des différences du nombre de copies sont détectées avec une résolution génomique d'aussi peu que 60 bp.

20. Procédé de la revendication 1, ladite étape de quantification incluant l'étape de détection de l'adresse spectrale dudit produit en utilisant la cytométrie en flux.

21. Procédé de la revendication 1, ladite sonde ayant une longueur comprise entre 50 et 2500 bases.

22. Procédé de la revendication 1, ladite matière particulaire ayant un diamètre compris entre 50 nm et 1 µm.

23. Procédé de détection d'une anomalie chromosomique suspectée dans l'acide nucléique génomique ou complémentaire dérivé du sujet, lequel procédé comprend un dosage d'hybridation de suspension, ledit procédé comprenant les étapes de :
la préparation d'une microsphère fluorescente spectralement codée ayant une première adresse spectrale,
l'identification d'une séquence de sonde d'acide nucléique cible génomique en vérifiant la séquence nucléotide-par-nucléotide d'une séquence d'acide nucléique cible dans laquelle on suspecte l'anomalie de résider,
la synthèse d'une sonde cible d'acide nucléique, spécifique pour une séquence cible génomique à faible nombre de copies, d'une séquence connue selon la séquence de sonde d'acide nucléique cible génomique identifiée,
la conjugaison de la sonde cible génomique à une microsphère ayant une première adresse spectrale,
la synthèse d'une sonde de référence sélectionnée pour l'hybridation à une séquence d'acide nucléique de référence ayant un nombre de copies connu de ladite séquence d'acide nucléique cible génomique,
la conjugaison de la sonde de référence à une microsphère ayant une deuxième adresse spectrale,
la réaction de la sonde cible avec une séquence cible chromosomique contenant l'anomalie, provoquant ainsi l'hybridation de la sonde cible à la séquence cible,
la réaction de la sonde de référence de séquence connue avec une séquence de référence chromosomique contenant ladite séquence cible chromosomique, provoquant ainsi l'hybridation de la sonde de référence à la séquence de référence,
la détection de la sonde cible hybridée pour vérifier l'existence de l'anomalie du chromosome ;
la quantification de la sonde cible hybridée détectée par comparaison de la réponse de la sonde cible hybridée détectée avec la réponse de la sonde de référence hybridée détectée,
sans présélection ou amplification de la séquence cible spécifique du locus,
dans lequel la séquence cible à faible nombre de copies a un nombre de copies de 10 ou moins.

24. Procédé de la revendication 23, ladite anomalie étant sélectionnée dans le groupe consistant en différences des nombres de copies de séquences dans ledit acide nucléique génomique ou complémentaire, duplications, délétions, inversions, transpositions, translocations et combinaisons de celles-ci.

25. Procédé de la revendication 23, ledit procédé détectant lesdites anomalies avec une résolution génomique d'aussi peu que 60 bp.

26. Procédé de la revendication 23, lesdites étapes de détection utilisant la cytométrie en flux.

27. Procédé de quantification génomique directe d'anomalies chromosomiques par détection par cytométrie en flux de séquences d'acide nucléique cible génomique marquées hybridées à des sondes d'acide nucléique génomique ou complémentaire conjuguées à des microsphères, spécifiques pour une séquence cible génomique à faible nombre de copies, de séquence connue sans présélection ou amplification de la séquence cible spécifique du locus, ledit procédé comprenant les étapes de :
la préparation d'une microsphère fluorescente spectralement codée ayant un diamètre compris entre 50 nm et 1 µm,
la synthèse d'une sonde cible, spécifique pour une séquence cible génomique à faible nombre de copies sélectionnée pour s'hybrider à une séquence d'acide nucléique particulière dans la cible dans laquelle l'anomalie est suspectée de résider,
la conjugaison de la sonde cible avec la microsphère,
l'hybridation de la sonde cible à une séquence d'acide nucléique cible,
le marquage de la séquence d'acide nucléique cible,
la détection de la sonde cible hybridée par cytométrie en flux, et
la quantification desdites anomalies sur la base des résultats de ladite cytométrie en flux,
dans lequel la séquence d'acide nucléique de la sonde est d'une longueur d'au moins 60 bases, et dans lequel ladite séquence cible à faible nombre de copies a un nombre de copies de 10 ou moins.

28. Procédé de la revendication 27, dans lequel la séquence d'acide nucléique d'une sonde est d'une longueur définie comprise entre 60 et 2500 bases.

29. Procédé de la revendication 27, ladite anomalie étant sélectionnée dans le groupe consistant en différences des nombres de copies de séquences dans ledit acide nucléique génomique ou complémentaire, duplications, délétions, inversions, transpositions, translocations et combinaisons de celles-ci.

30. Procédé de la revendication 27, ledit procédé détectant lesdites anomalies avec une résolution génomique d'au peu que 60 bp.

31. Procédé de détection d'anomalies chromosomiques sans présélection ou amplification de la séquence cible spécifique du locus, ledit procédé comprenant les étapes de :
la préparation d'une sonde d'hybridation de séquence connue par couplage d'une microsphère de polystyrène spectralement codée avec une séquence d'ADN synthétique complémentaire d'une séquence cible génomique à faible nombre de copies,
l'hybridation de ladite sonde à l'ADN génomique,
le marquage de l'ADN génomique,
la détection du produit de ladite hybridation par cytométrie en flux, dans lequel la séquence d'acide nucléique de la sonde est d'une longueur d'au moins 60 bases, et dans lequel ladite séquence cible à faible nombre de copies a un nombre de copies de 10 ou moins.

32. Procédé de la revendication 31, ladite anomalie étant sélectionnée dans le groupe consistant en différences des nombres de copies de séquences dans ledit acide nucléique génomique ou complémentaire, duplications, délétions, inversions, transpositions, translocations et combinaisons de celles-ci.

33. Procédé de la revendication 31, ledit procédé détectant lesdites anomalies avec une résolution génomique d'aussi peu que 60 bp.

34. Procédé de la revendication 31, comprenant en outre l'étape de comparaison des résultats de ladite cytométrie en flux avec les résultats de cytométrie en flux d'un produit d'hybridation ayant un nombre de copies connu correspondant à ladite anomalie.

35. Procédé de la revendication 31, dans lequel ladite sonde est complémentaire de 10 emplacements ou moins dans l'ADN génomique.

36. Procédé de la revendication 31, l'ADN étant non amplifié.

37. Procédé de la revendication 1, dans lequel ladite sonde est de séquence connue avant hybridation.

38. Procédé de la revendication 1, dans lequel les conditions dans lesquelles l'hybridation a lieu sont adaptées à la séquence particulière de la sonde.

39. Procédé de la revendication 38, dans lequel le point de fusion de la sonde est connu avant hybridation.

40. Procédé de la revendication 1, dans lequel ledit génome est le génome humain.

41. Procédé de la revendication 1, dans lequel la teneur G-C de la sonde est connue avant hybridation.

42. Procédé de la revendication 1, dans lequel il y a hybridation spécifique à ladite séquence d'acide nucléique cible.

43. Procédé de la revendication 1, dans lequel la séquence de l'acide nucléique cible est connue avant hybridation.

44. Procédé de la revendication 1, comprenant en outre un dosage de suspension en trois dimensions.

45. Procédé de la revendication 1, dans lequel ladite hybridation de ladite sonde est le résultat d'une réaction d'hybridation.


### SEQUENCE LISTING

<110> Children's Mercy Hospital
   Newkirk, Heather
   Rogan, Peter
   Knoll, Joan
<120> QUANTIFICATION OF MICROSPHERE SUSPENSION HYBRIDIZATION AND USES THEREOF
<130> 36401-PCT
<150> 60/708,734
   <151> 2005-08-16
<160> 28
<170> PatentIn version 3.3
<210> 1
   <211> 2287
   <212> DNA
   <213> Homo sapiens
<400> 1
   aatagatgca atgggggtga gagagaatgt tcacttgcca agggaatgag ggcagacaag     60
   agaaaggaaa attgagatgg gaggaggtga cctgcagagg aattgaagag aaagaaatta    120
   ttgaggtaag agcaagctta tttctttcta ttttactttt tcggggagaa gatttgcaga    180
   tgcttcatcc tttcatttta agatgtcttc cacagacgct aagaataact tgtctggagg    240
   aatctagggc ttgtcttcta accccaaatt tgaacaaggc ctgtatctga tctggcagcc    300
   ctgcttctga gagacttgag acttgttcac cttccacctt ttcctcatcc cggggagaat    360
   cttctcaccc catcctttgc cttcataatt aagggaacaa tttgaggtct aaaggaagaa    420
   gtgaatttca aatcagttta ataaaacagc acaaatctgg gaattgagga atgcattccc    480
   tttataacag ctgaagtcac caacaatcta ttattcatga cagcaagcaa agactgaggc    540
   aaaagtgccc taagttggct gggtgcagtc ccagcacttt ggaaggccaa ggcaagagga    600
   tcacttgagc ccaggacttc gagaccagcc tgggcaagat ggtgagacca catctctaca    660
   aaaataaata aatacaaaaa ttagctgggt gtggtggtgc ctccctgaag tcccagctgc    720
   tcagaaggga ggatggcttg agcccaggag ttcaaggctg caatgagcta tgaccacacc    780
   actgcactct agcctgggtg acagagaacc tgtctctatt taaaaaaaaa aaaaaagtgc    840
   ctcaagttat actgtcaatt gcagtgattg cctcagcttt ccaaattcac tatgctcccc    900
   aattcatccc tgagtcatgc caccttctct tgtgttagca gggacattaa aacttgccca    960
   aattcctgct ggctctccca gccccagcag aaaagactgg gagaaccaag caactttgct   1020
   ccctaggttc ccaatctcct cctcctccct aaaagaagct tcgctgactg gaataaccta   1080
   tggcccatag gatttttggt ggaccaaatt aaactcacca gagagggtct gggtggaccc   1140
   aacaccaaaa tgaaataaag ttgatgtcct agtttgtatg atctgcttgt acataaaaga   1200
   gagcaaggag aacatagaca tgtttggaac tgaagttatc tttgacagtt ttcactcctt   1260
   atcacactga gaatgcaggc aaggaaacta tctaaccaga atggggatat attagaagtt   1320
   taacctcaga ttctggtcct ttctgtgaag gaagctgctt cagctggggg cttggcagga 1380
   gcaatctaaa ttctctcatt agaccttaaa tgtctctatc tgctctcccc agcccaaatc 1440
   tagggaggcc aaacccaaga tcaaaggttg gggctaggcc agtgaggtgg ctcatgcctg 1500
   taatcccagc actttgggag gccaaggagg gcagatcacc tgaggtcggg agttcaagac 1560
   cagcctgacc aacatggtga aacccccatc tctactaaaa aatacaaaaa ttagccgggt 1620
   gtggtggcag gcacctataa tcccagctac tcaagaggct gaagcaggag aatcgcttga 1680
   acccgggagg cggaggttgc agtgagctga gatcgcacca ttgcactcca gcctgggcga 1740
   cagagcaaga ctcaaaaaaa aaaaaaaaaa gttggggctt aggaagtaaa gaatattcat 1800
   aattttttcc ttctttcttt cttttttttt caggcagggt ttcgctctgt tgccccaggc 1860
   tagagtgcaa tggtgtaatc acggctccct acagctttga cctcctgggc tcaagcgatc 1920
   ctcccacctc agcctcctga gtaggtagga ctacaagcat gcaccaccac aagtttttca 1980
   ctatgagatg aattgttttt gttgctgtta gagaaaggat atttctttta atgcaagcta 2040
   attaaactgt cataaatctg gttgagaacc ttagactgta tgtagtacat attgtaatgg 2100
   gattagtgct agctatgtgg gccagaatag ttgggttcaa gcccttgctt ggccattttt 2160
   tttttttttt tttaatacag ggtctcactt tgtcacccag gctggagtgc agtggcgcaa 2220
   tctcggcccg ctgcaacctc tgcctcccag gcccaagcaa tcctcccacc tcaacctccc 2280
   aagtagc 2287
<210> 2
   <211> 1344
   <212> DNA
   <213> Homo sapiens
<400> 2
   cggttctgga accaaatctt gacctgtgtt tcattgagct ccagggtggc ggcaatctcc      60
   accctccggg cccggctcag gtacttgttg aaatggaact ccttttccag ttctgtcagc     120
   tgccttgtgg tgaagttggt gcggaggcca ctgggcgagc ccaggcctgg ctctgacacc     180
   ttcgctaggg gcggggcagg gagaaaggcc aggtcaattc tctcacctct ttctccttcc     240
   gcttccctcc tcccggggct ggctctggac ctccatttgc cattctgccc aagtacagtt     300
   gtcaaagttc ccagggacca ccaggtaccc ccatggtgat caccaagtct ggagcagcct     360
   tccccatact tccatccccc atgcacacca ggtcctgggt ctgtgctggg tgcacagatc     420
   aggaagtggg gtgtgtatgg aaacttactc ctggggtgac cggttacata ctgggtgggg     480
   agacctcacc tgacctgaga cgtaggttgt gctcttacct gtgtctacca gagccgctga     540
   aagagaagaa cccagcccag acccaggaca tgtcactgca ggggaagcag agatgctttg     600
   ggccccggag aaggggtggc cacatctgag ccctacctgt cttgggtggg tttctcttaa     660
   ccttcatcca gtcgaaggtc cgggccgtgg gggtgttagg ttctgaaggg cagggtgttt     720
   ccttgtcctc ggagaggaga tcagcatagg ccggtgcaaa gctcgcggtc tgctcgttcc     780
   cataaggggg atgctgcgga ggatatggcc ccggaccggc tccacctgct ccgtagccat     840
   cggacaagcc ccctagctgg gccccgtagc tcgagggatg aaaatagcct ccgtctcctt     900
   ctgattgacc cagagggtag tactgagaag gcccgtagct ggggctgcag gcggcaggag     960
   cataccccga gggcgcggag ctggggaagg gcacccccag ggtcgaaggc ggctgctggg    1020
   cgggatagcc ggagttctgc tgaaacgcag ggctggacag ccccccaccg tagcggccct    1080
   cgcttgcata gctgtcaacc gcctgagccg agcttggggg aaaggaggtt ggggcgctgt    1140
   gggcgctgta ggcgctgggt ccccggttac agagtgggta ctctaagaag gagttcatcc    1200
   tattatagtc catgcgtcaa ggccgcagga gggtcggccc gtttggggga gacaccctct    1260
   tgccctacaa cctttcggca gtatgtcaca gcgctggggc tcgaatccat ggcctgaccc    1320
   gctcgcctgg gcaagcgctt ccct                                           1344
<210> 3
   <211> 94
   <212> DNA
   <213> Homo sapiens
<400> 3
   ttatcctctc tctagccatc catagctgta gctggctcac tcactcagac ttaaataact      60
   ccctatatct ctctagagag ccacaatgag caca                                  94
<210> 4
   <211> 2304
   <212> DNA
   <213> Homo sapiens
<400> 4
   gtggcttatg cctgtaattt cacactttgg gaggccaaag gcaggtggat tgcttgagcc     60
   caggagttca agatcatcct gggcaacata agaagaccct gtctctacaa caagaacaaa    120
   agaaattagc cagacgtgtt atcacacctg tgttcccagc tacttgagtg gctgaggtgg    180
   gagggtcact tgagctcggg aggccaatgc tgcagtgagc catgatcacg ccactgcact    240
   gcagcctggg taacagagca agaccccatc tcaaaacaaa gtttgagaac tgttgctatg    300
   gaaagtctat caagaaggca catagggatg tttgttgctg aagtactaac actgaatgat    360
   gtctgtgttg tggtgagtag aaaaaaagca atttgaaaat cattatttat ggtatggccc    420
   catttttgta aaacaaaaat atctgtttat ttgtgtatgt ccaggaaaaa aaagtatgga    480
   aggattttac accgaaattt ttatctctag ggacttggag ctttaggtgg aatcggagaa    540
   aactttctct ttttggtttg tgtgatgttc caagtgtctt tttacaacaa acttgggtta    600
   tcttataata cgaaaaattc gaagatttaa aaactgaggc caggcacggt ggctcatgcc    660
   tataatccca gcactttggg aggttgacac atgtggatca cctgaggtca ggagtttgag    720
   accagcttga ccaagaaggt aaaaccctgt ctctactaaa aatacaaaaa ttagctgggc    780
   gtggttgcag gtgcctgtag tcccagctac tcgggaggct gagacaggag agttgcttag    840
   acccaggagg tcgaggttgc aacgagctga gattgcacgc cactgcactc ccagcctggg    900
   cgacggagtg agactccatc tcaaaataaa taaataaata aaaataaaaa ctgaattgat     960
   gacagcccaa cctgtcactt ttttcagatc cctttttatg aaagaatttg cttaagttgt   1020
   gtctgaagac aaaccaattt ctttggaccc tgggtattct ttttctaagg gaataccatg   1080
   ttattttgtg ttacagattg tttgcgatct ttcataggct gatctttcta gagttggtta   1140
   atatccatgt aggttagatt gaaaaacttg aattcagaaa tgtacggtgt tggagcagac   1200
   atggatctgg aagccaagaa tagcgttggt gttgttgttg atggtgaatc tgaaagagtg   1260
   ggcagatggc aactttttgc cagaatcatt cagggtcaaa gggctagtca tatttacaag   1320
   ggaaaagagg aaaccatgtt attaaatatt tcatgcacca tatgttcctt tcaaatttca   1380
   gtggcttgac taagaaaatt cacttttggg agagttggta caacaattga aagcacttag   1440
   tagtcaactt tttccttagt gtttccttct tccttcaaca aacatttatt ttattgagca   1500
   cagactatga caggtcgtgt tgtagtcact gatgatatat ttgtaaacaa aacaatccct   1560
   gccctcctag aacttacatt ctagtgggag gagacacatc ataaataagc taagtaaata   1620
   aaatacagat atgttagtga tatgtgctga ggagaagaat aacatagaga aggggacagg   1680
   gaatggggta ggaggtgtaa ttttagttag ggtggctaga gaggcctgac tgagagggcg   1740
   gcatttgatt aaagacccga aggaggaaga tgagcagcag gccctgttga aatatgtgaa   1800
   agaatttcag gccgagggaa ccaggatgca gaaacctgag gcagagcttg ctagtttcaa   1860
   ggagcagcct agaggccact gcaccagagc aaagagcaaa aggagagatg caggaggtaa   1920
   aatggagggt gggaagagaa tggctgaagg agagggtggc cttattggcc catttcaagg   1980
   attttgactt tgattcatat gatgtgggaa ggccttggag aattttccga ggggactata   2040
   ctatctgatg tatgtttcag tatgaaatag aatctctctg gctgctatga tctgaaggag   2100
   gacaagggag cagggattcc acgggggcat tgattgcaca gagccaggta agagtgatga   2160
   tgcttggacc aaagtggtgg cactgggtgg agggtcagaa ccgacaagat ttgctctcgg   2220
   attagatgtg ggatatgggg gtgtgagagg ggccaagtgt gactctcagg ttttcgaact   2280
   gaataactgg aagaatagag ttgc                                           2304
<210> 5
   <211> 101
   <212> DNA
   <213> Homo sapiens
<400> 5
   gatctttcta gagttggtta atatccatgt aggttagatt gaaaaacttg aattcagaaa     60
   tgtacggtgt tggagcagac atggatctgg aagccaagaa t                        101
<210> 6
   <211> 1383
   <212> DNA
   <213> Homo sapiens
<400> 6
   atttggaaag attatatcca tctacttaat gctagggatt ggattgtggc acgtggactg     60
   tgaaaatgca aaattggagg tatgtacttt attttagaga cagggtcttg ctttgtcacc    120
   caggctggag tgtagtggca cagtcatagc tcactgcagc cttgaactcc tgggctccag    180
   tgatcctctt gccttagcct cccaaagcaa tgggatcgta caggtgagcc attgcacctg    240
   gccatgtatg tacatttttt ataagcattc agttgtttcc aatagtctaa tacattttgt    300
   atggccttct tatttttcat gaaaccttac tacccatttt aagaaatttg taatatagaa    360
   gaaaacagaa gtttcaattg tggggtaagt aaagaaagat gatcaaagat gatttgacca    420
   aaagagtttt acttcattta aatgaagcat gttgtcaaag aaaatgaaag tgggaaaaaa    480
   tgtgaaatca ttttgtatca gttcaggaca gaaccgtgct tgtgcttcag atctccaagt    540
   gtgaacacac tgatgtgttg tgaagtgtgt tgcaaataga agcatttttt gaatgggagg    600
   gagagggatg tgttaacatg atttagctgg gaattaaacc cataggaatg tgtcgggcat    660
   attttctgtg actagggggt aaaaagatta aaatggctac tgaggatggg cttcagtaag    720
   aggaggtctc ccgaaggcct tctctgccct gtgcctctgc agttccctga ctccttgctg    780
   tgttcgtccc tgcctttttg gtagttgtgc ccctgatgtc actcccctgt gtgttcagat    840
   cctacctgct ggccaggtgc agtggctcac acctgtaatc ccagcacttt gggaacccaa    900
   ggtgggacgg tcctctgaga ccaggagttt gagagagtgg cctggacaac atagcaacac    960
   cctattagta ctaaaaataa aaaagttagc taggcataat aatgtcctgt gcttgtctag   1020
   ctacttggga ggctgaggca gaaggattac ttgagtccag gagttcgagg ctgaagcaaa   1080
   ctgtgatcat atcattgtac tccagcctgt gtgataaagc aagaccctat cctttaaaag   1140
   taaaaaacaa acaaacaaac aaaaaacaaa cctgcttaac ctttcaacac tctcttccag   1200
   tcccccctcc cttctgaaac catattttac atctctggtc ctcattaaat tttctgtaac   1260
   ttctgccctt gtaactcttc tgcaacacaa atgaatacat aatcatatac ttcctagtag   1320
   tatttcctaa tagttttatg tgagtttcat ggaagagagt gttgaaacgt aggtaggttt   1380
   gtt                                                                 1383
<210> 7
   <211> 102
   <212> DNA
   <213> Homo sapiens
<400> 7
   atttggaaag attatatcca tctacttaat gctagggatt ggattgtggc acgtggactg     60
   tgaaaatgca aaattggagg tatgtacttt attttagaga ca                      102
<210> 8
   <211> 99
   <212> DNA
   <213> Homo sapiens
<400> 8
   accaactttc atgccctagg tgaaacggag gacgtcttgg gaggtagaga gttgcccaag    60
   gtctgaatcc atgggatcta ctaagaacaa gacccagac                           99
<210> 9
   <211> 102
   <212> DNA
   <213> Homo sapiens
<400> 9
   agggcagctc atgacatgcc tccatgtttt ccatgtgggt catcaaacaa cagagcacca    60
   gagagggttg aactaaatga atactcaagt gccttccagt cc                      102
<210> 10
   <211> 81
   <212> DNA
   <213> Homo sapiens
<400> 10
   aaaggcgatc tcctcctgct cctgctagag cgcagcgagc gcacggcgtt tccgcagtaa    60
   tcctgagcac ggcaaggacc c                                               81
<210> 11
   <211> 81
   <212> DNA
   <213> Homo sapiens
<400> 11
   attcatacca gcaacagcag tggcatgatg cgggcaggat tgccagtgtc cgtgcatgcg    60
   tttgcactgg tgattgcagt g                                               81
<210> 12
   <211> 81
   <212> DNA
   <213> Homo sapiens
<400> 12
   gcaatagtgg tggacaagtt ggcacctcga ttgtctcagc tcaaggttct gcacaaagtg    60
   tttagtactg gagtagcaga a                                               81
<210> 13
   <211> 81
   <212> DNA
   <213> Homo sapiens
<400> 13
   tacccaccac cctctaacca ccaaatggtc tatcctagat ggtgaagttg ggttaattgt 60
   tagacacggt ggaatcttta t 81
<210> 14
   <211> 81
   <212> DNA
   <213> Homo sapiens
<400> 14
   cggcagctag gggcaggagt gcaagaatga tgtatccttt caagtatcat gaccatcggg 60
   gtgaattgct gagttctggg g 81
<210> 15
   <211> 81
   <212> DNA
   <213> Homo sapiens
<400> 15
   taagtccttg gcattactta aagaaggcca ttagatatat gtcacgtgtg agagtgaggt 60
   ttgcagctgg gaagtaagcc t 81
<210> 16
   <211> 81
   <212> DNA
   <213> Homo sapiens
<400> 16
   gaaacagtca tcgagtggga tgacaacttg gaaaggcttt gtgggccttt gtgacctcat 60
   ggtatgcatc ccctcagggt g 81
<210> 17
   <211> 81
   <212> DNA
   <213> Homo sapiens
<400> 17
   tggcgtctgc taggagtctc ctatccggcg tttgtgccgc agaaaacctt taacctgcgt 60
   tttacccgta aacattttcc t 81
<210> 18
   <211> 81
   <212> DNA
   <213> Homo sapiens
<400> 18
   aaaagcataa gttttagtca catcaaaata taaacaatct tgaaattaat tcataagtaa 60
   atcaatagat atcttgaaat t 81
<210> 19
   <211> 81
   <212> DNA
   <213> Homo sapiens
<400> 19
   ttcggagagt tacccaggtg tagtaccctt ccaagtagat aatgattgaa agagtatgtg 60
   ctggaggtgt atgatatttt c 81
<210> 20
   <211> 81
   <212> DNA
   <213> Homo sapiens
<400> 20
   ggttttgatt ttgatatact ccagtttatc tatttttgtg tttccttagc ttttggtgtt 60
   gtagtcaaga aatcattgcc a 81
<210> 21
   <211> 81
   <212> DNA
   <213> Homo sapiens
<400> 21
   tgcattcttt ttaaaaaata tttttagtct ttacatttca gaatcagtat ttcaaatgtg 60
   acttgtagga ctacgtgcag t 81
<210> 22
   <211> 81
   <212> DNA
   <213> Homo sapiens
<400> 22
   cttgctgttg tgccgttctg ccccgatggt atcctgtccg ctcgcattgg ggcgcgtccc 60
   ccatccgccc ccaactgtgg t 81
<210> 23
   <211> 81
   <212> DNA
   <213> Homo sapiens
<400> 23
   ggttactgat tcataggggt atgtcgaggg cttttgtctg gaattccctg cttaccagta 60
   gatgattggt ttacatattt t 81
<210> 24
   <211> 81
   <212> DNA
   <213> Homo sapiens
<400> 24
   ggccgcagag gcaggctggc gcgcatgctc aggcggggat gtgtgcgaag cctgccgctg 60
   ctgcagcgag tctggcgcag a 81
<210> 25
   <211> 81
   <212> DNA
   <213> Homo sapiens
<400> 25
   agtatttcaa atgtgacttg taggactacg tgcagtgagg aagattgtta atcatcctat 60
   tctgaccagg agttctgaca a 81
<210> 26
   <211> 81
   <212> DNA
   <213> Homo sapiens
<400> 26
   caaacactgg gacccagaat agtaccctgt ggtcatagtg cccacagttc actaagcacc 60
   ctcacaggtc tttgacagaa c 81
<210> 27
   <211> 81
   <212> DNA
   <213> Homo sapiens
<400> 27
   tttacctgcg cacttgcaga tctttctcca ggagtgagtt taaggtccgc acctccgctc 60
   tggatggtga gcggagcaca g 81
<210> 28
   <211> 81
   <212> DNA
   <213> Homo sapiens
<400> 28
   aagacacttc aatcacccgc agcccatcca tctgagagcg acatggaagc gtcaaaaaca 60
   tctttcttca aagacttttg g 81